# EUROPEAN PATENT SPECIFICATION

(11) **EP 3 380 601 B1**
(45) Date of publication and mention of the grant of the patent: **13.07.2022**
(21) Application number: 16804859.3
(22) Date of filing: 23.11.2016
(51) Int. Cl.: C12N 5/00

(54) **MICROSCAFFOLD**
MIKROGERÜST
MICRO-ÉCHAFAUDAGE

(30) Priority: 24.11.2015 GB 201520737
(43) Date of publication of application: 03.10.2018
(73) Proprietor: The Electrospinning Company Ltd, Didcot OX11 0RL (GB)
(72) Inventor: MCKEAN, Robert James, Didcot Oxfordshire OX11 0RL (GB); ALLENBY, Gary, Loughborough Leicestershire LE12 5AP (GB); FABRE DE LA RIPELLE, Erik Léon Victor, Bristol BS3 4HX (GB)
(74) Representative: Icely, Dominic Michael
(86) International application number: PCT/GB2016/053679
(87) International publication number: WO 2017/089797

(56) References cited:
- Caroline Dang ET AL: "Aligning poly(L-lactic acid) nanofibers with magnetic Fe 3 O 4 nanoparticles using modified electrospinning methods", Journal of Undergraduate Research in Bioengineering, 1 January 2007 (2007-01-01), XP055340574, Retrieved from the Internet: URL:https://www.uweb.engr.washington.edu/e ducation/pdf/Dang2007.pdf [retrieved on 2017-01-31]
- SAVVA I ET AL: "Fabrication, characterization, and evaluation in drug release properties of magnetoactive poly(ethylene oxide)-poly(l -lactide) electrospun membranes", BIOMACROMOLECULES, AMERICAN CHEMICAL SOCIETY, US, vol. 14, no. 12, 9 December 2013 (2013-12-09), pages 4436-4446, XP002765264, ISSN: 1525-7797
- Hsin-Ta Wang ET AL: "Development and biocompatibility tests of electrospun poly-l-lactide nanofibrous membranes incorporating oleic acid-coated Fe3O4", Journal of Polymer Engineering, 22 November 2013 (2013-11-22), pages 241-245, XP055340990, DOI: 10.1515/polyeng-2013-0206 Retrieved from the Internet: URL:https://www.degruyter.com/downloadpdf/ j/polyeng.2014.34.issue-3/polyeng-2013-020 6/polyeng-2013-0206.xml [retrieved on 2017-02-01]

## Description

### FIELD OF THE INVENTION

The invention relates to a scaffold suitable for supporting cell growth.

### BACKGROUND TO THE INVENTION

In the drug discovery process, mammalian cells are routinely cultured in industry-standard multi-well plates, which fit a range of standard measuring instruments and automated (robotic) processing equipment. This includes, for example, equipment for rapidly dispensing liquid in and out of the plates, equipment for rapidly measuring a signal (such as fluorescence, luminescence or optical absorbance), and equipment for storing and moving the plates. Cellular responses to drug stimulation are typically determined using colorimetric, fluorescence and bioluminescence based assays, and readouts are commonly obtained using microplate readers and automated confocal microscopy.

Such cell-based assays are routinely used in high throughput screening (HTS), which is commonly used by pharmaceutical companies to assess the efficacy and toxicity of drug candidates. Simple assay readouts are used to determine the level of drug required to induce apoptosis, inhibit cell proliferation or decrease cell viability. Drug candidates are currently screened in two-dimensional cultures of cells (2D cell cultures) in 96, 384 or 1536 well plates and assays are designed to be highly efficient and low cost due to the high number of potentially active compounds to be screened. High content screening (HCS) is also commonly used by pharmaceutical companies, to explore the action of a candidate drug on a cell in more detail, by carrying out multiple assays in a single well and using more complex analytical instruments such as confocal microscopy. Targets are commonly screened in 2D on 96 well plates.

In both HTS and HCS candidate drugs are typically screened in two-dimensional cultures of cells on the flat base of each well. Cells cultured in 2D on tissue culture plastic are flat, have 50% of their surface area exposed to tissue culture plastic, and 50% of their cell surface area exposed directly to cell culture media. This inhibits the production of extracellular matrix (ECM) which is responsible for signalling between cells over long distances and results in tissue specific gene expression. As a result, cells cultured in 2D are not genetically or phenotypically similar to their *in vivo* counterparts found in tissues, which comprise both cells and matrix molecules. Screening in 2D cell cultures can therefore lead to a high number of false positive results, which increases the number of drugs that fail only after expensive animal trials. Additionally, pre-clinical testing of novel compounds in model organisms such as mice and rats does not accurately represent how a drug may interact with cells in the more complex human physiological system. There is therefore an ongoing need to develop improved assay methods and equipment which facilitate more accurate *in vitro* drug discovery, and in particular which can reduce the number of false positives at an early stage.

Another difficulty lies in the fact that most mammalian cells are anchorage-dependent and have to be cultured on a suitable substrate that is specifically treated to allow cell adhesion and spreading. Accordingly, when such cells are cultured in a multi-well plate they become anchored to the tissue culture plastic at the base of each well, making it impossible to manipulate the cell cultures thereafter. Such adherent cultures cannot for example be transferred in and out of the wells in a plate, or from the wells in one plate to another, and this greatly limits flexibility in the drug screening process. It would be desirable to have a system where cell cultures could be transferred rapidly in and out of sample wells and from one location to another, using industry standard robotic processing equipment. This would not only increase the ease and speed with which cell cultures can be handled and manipulated during the drug screening process, allowing for greater efficiency, it would also allow for the development of more complex assays and screening procedures for a wide variety of adherent cell types. To increase the ease with which cell cultures may be manipulated would also be valuable in other related fields, including for instance regenerative medicine and tissue engineering.

Improved equipment and methodology is therefore needed which not only facilitates more accurate *in vitro* drug discovery but which also allows for greater flexiblility in terms of cell culture manipulation, e.g. using automated processing equipment of the kind commonly employed in drug screening.

Dang et al. (Journal of Undergraduate Research in Bioengineering, 2017) discloses the preparation and use of PLLA fibres by electrospinning comprising magnetic particles for cell culture applications, including culture of Schwann and PC12 cells.

Savva et al. (Biomacromolecules, 2013, 14, 12, 4436-4444) discloses electrospun PLLA fibres and oleic acid-coated magnetite nanoparticles which are biocompatible.

Wang et al. (J. Polym. Eng., 2013, 241-5) discloses an electrospun PLLA nanofibrous scaffold incorporating oleic acid coated magnetite which resembles an extracellular matrix and can be used to support cells, e.g. osteoblasts.

### SUMMARY OF THE INVENTION

The present invention provides microscaffold particles which facilitate the growth of three-dimensional (3D) cell cultures with a high degree of consistency and reproducibility and are capable of supporting 3D culture growth for a wide variety of cells. The inventors have shown that various mammalian cell types, including hepatocytes, kidney cells and neuroblastoma cells, successfully attach to the microscaffolds, that they remain viable after attachment, and that they respond in a pharmacological manner to chemical stimulation. Moreover, the cells in 3D culture on the microscaffolds have 100% of their cell surface area exposed to other cells and matrix. This stimulates specific signaling pathways to initiate tissue-specific gene expression and stimulate the production of extracellular matrix (ECM). As a result, the cultures grown in the microscaffolds of the invention are more similar to cells found within tissues in the body and provide a more accurate *in vitro* model for drug discovery than cells grown in 2D. Such 3D cell cultures are also more resistant to drug treatment than cells grown in 2D, meaning that the microscaffolds of the invention can advantageously reduce the number of false positives at the high throughput screening (HTS) and high content screening (HCS) stages and thereby reduce the number of drugs that fail only after undergoing animal trials. The invention therefore has applications in the 3Rs to reduce, refine and replace animal models with more accurate *in vitro* human tissue models. Furthermore, animal studies on model systems such as mice and rats do not accurately represent the more complex human physiological environment for which the drug is ultimately intended.

At the same time, the small size of the microscaffolds of the invention means that cell-laden microscaffolds can be readily suspended in culture medium, so that the sample resembles a suspension culture more than a stationary cell culture adhered to a surface. This in turn provides advantages, including that such cultures are more uniformly bathed in the nutrient-containing culture medium, and also that external manipulation of the culture is possible, meaning that handling of the cell cultures is rendered easy. Thus, in a multi-well plate, 3D cell cultures grown in the microscaffolds of the invention may be suspended in culture medium in each well, rather than being anchored to the base of the well, meaning that cell cultures may easily be manipulated by automated processing equipment, including robotic pipetting devices for dispensing liquid in and out of plate wells. Cell cultures in microscaffolds of the invention can therefore be transferred rapidly in and out of sample wells and from one location to another, using industry standard processing equipment, allowing increases in the ease and speed with which cultures can be handled and manipulated during drug screening, in turn rendering the process more efficient and cost-effective, and allowing for development of more complex assays and screening procedures for a wide variety of cell types. A further surprising advantage of the microscaffolds of the invention is that mammalian cell cultures grown in the microscaffolds may be cryopreserved in freezing solution for substantial periods of time, and then thawed again before use, without adversely affecting cell viability or the ability of the cells to respond to chemical stimulation in a pharmacological manner. Cells cultures grown in the scaffolds may therefore be stored, in a frozen state, before they are employed by the end-user in drug testing or other applications, without adverse effect.

Accordingly, the invention provides a microscaffold comprising a porous particle, which particle: comprises a three dimensional network of electrospun fibres, which fibres comprise a biocompatible polymer, wherein the particle has the shape of a cylinder or a polygonal prism, wherein the diameter of the cylinder or polygonal prism is from 20 µm to 2000 µm, and wherein the height of the cylinder or polygonal prism is from 10 µm to 200 µm.

The inventors have additionally found that magnetic material may be incorporated into the microscaffold, without adversely affecting the viability of cells grown in the microscaffold or the ability of the cell cultures to respond pharmacologically to chemical stimuli, or indeed the ability of the cell-laden scaffolds to be cryopreserved. Advantageously, the presence in the scaffold of a magnetic material increases the ease with which the microscaffolds may be maintained in suspension and manipulated externally. Such magnetic, cell-laden microscaffolds can be maintained in suspension by stirring using a standard magnetic stirring device. In addition, the magnetic microscaffolds may be manipulated using one or more external magnets, allowing for magnetic transfer from one location to another location, or indeed for retaining the microscaffolds in a particular position whilst the cell medium is renewed or replenished.

Accorgingly, in one preferred embodiment of the microscaffold of the invention, the microscaffold comprises said porous particle, wherein the particle: comprises said three dimensional network of electrospun fibres, which fibres comprise a biocompatible polymer; wherein the particle has the shape of a cylinder or a polygonal prism, wherein the diameter of the cylinder or polygonal prism is from 20 µm to 2000 µm, and wherein the height of the cylinder or polygonal prism is from 10 µm to 200 µm; and further comprises a magnetic material. Usually, the fibres further comprise the magnetic material. The magnetic material is typically a paramagnetic, ferromagnetic, ferrimagnetic or superparamagnetic material. It may, for instance, be iron (II, III) oxide.

The invention also provides a process for producing a microscaffold, which process comprises:
(a) producing a scaffold layer, which scaffold layer comprises a porous, three dimensional network of electrospun fibres, which fibres comprise a biocompatible polymer; and
(b) cutting the scaffold layer to produce a porous particle, which particle comprises said three dimensional network of fibres and wherein the particle is cut such that it has the shape of cylinder or a polygonal prism, wherein the diameter of the cylinder or polygonal prism is from 20 µm to 2000 µm, and wherein the height of the cylinder or polygonal prism is from 10 µm to 200 µm.

The invention also provides a microscaffold which is obtainable by, or obtained by, the process of the invention for producing a microscaffold.

The invention also provides a composition which comprises a microscaffold of the invention, or a plurality of microscaffolds of the invention.

In another aspect, the invention provides a multi-well assay plate comprising: a plurality of sample wells; and a composition of the invention in at least one of the sample wells.

Herein also is provided the use not according to the invention of a microscaffold of the invention as defined above, a composition of the invention as defined above, or a multi-well assay plate of the invention as defined above, in: regenerative medicine, tissue engineering, screening compounds for biological use or drug screening.

In a further aspect, the invention provides a method of manipulating one or more microscaffolds, which method comprises exposing a composition which comprises one or more microscaffolds of the invention as defined above which further comprise a magnetic material, to a magnetic field of a magnet, and thereby causing the one or more microscaffolds in the composition to be attracted to said magnet by magnetic attraction.

The invention further provides a process for producing a microscaffold having cells and/or one or more reagents disposed on a surface thereof, which process comprises disposing a bioink onto a surface of a microscaffold of the invention as defined above, wherein the bioink is a composition which comprises cells and/or one or more reagents.

The invention further provides a bioink, which bioink comprises a microscaffold of the invention as defined herein. The microscaffold in the bioink generally further comprises cells attached to the microscaffold and, optionally, extracellular matrix.

In another aspect, the invention provides a method of providing a cell culture in one or more of the wells of a multi-well plate, the method comprising printing a bioink of the invention as defined above into one or more wells of a multi-well plate.

The invention also provides a process for producing a tissue construct, which process comprises printing a bioink of the invention as defined above to produce the tissue construct.

### BRIEF DESCRIPTION OF THE FIGURES

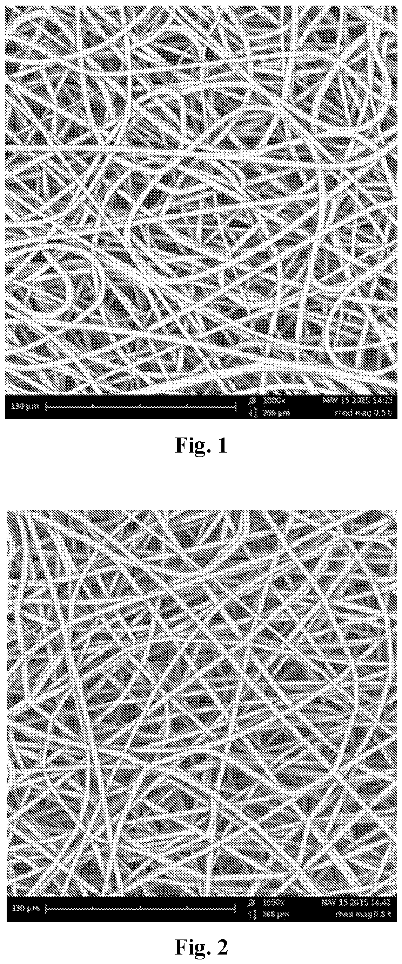

Fig. 1 is a scanning electron microscopy (SEM) image (1000× magnification) of the bottom of a scaffold produced by electrospinning.
Fig. 2 is an SEM image (1000x magnification) of the top of the electrospun scaffold shown in Fig. 1.

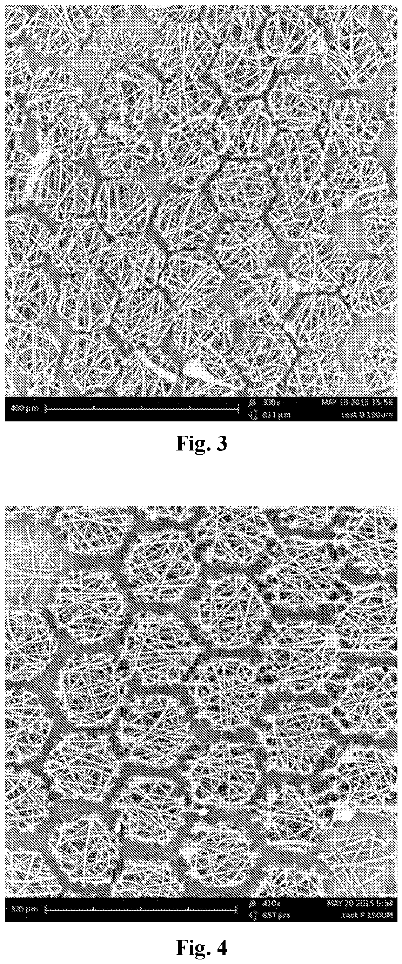

Fig. 3 is an SEM image of microscaffolds of the invention produced by laser machining an electrospun scaffold at focus+100 µm.
Fig. 4 is an SEM image of microscaffolds of the invention produced by laser machining an electrospun scaffold at focus-150 µm.

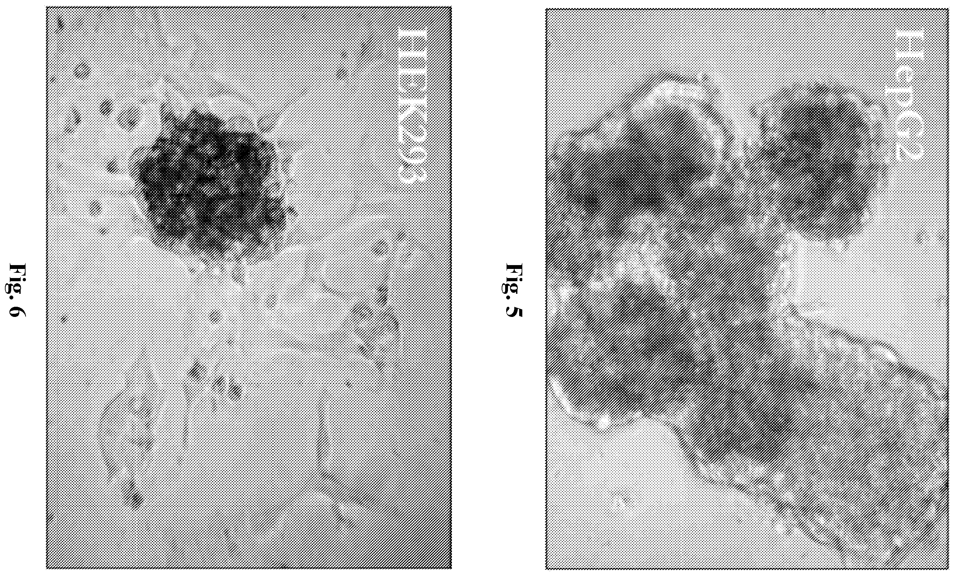

Fig. 5 is a light microscope image of HepG2 hepatocytes adhered to a number of microscaffolds of the invention after 4 days' growth.
Fig. 6 is a light microscope image of HEK293 kidney cells adhered to a microscaffold of the invention after 4 days' growth.

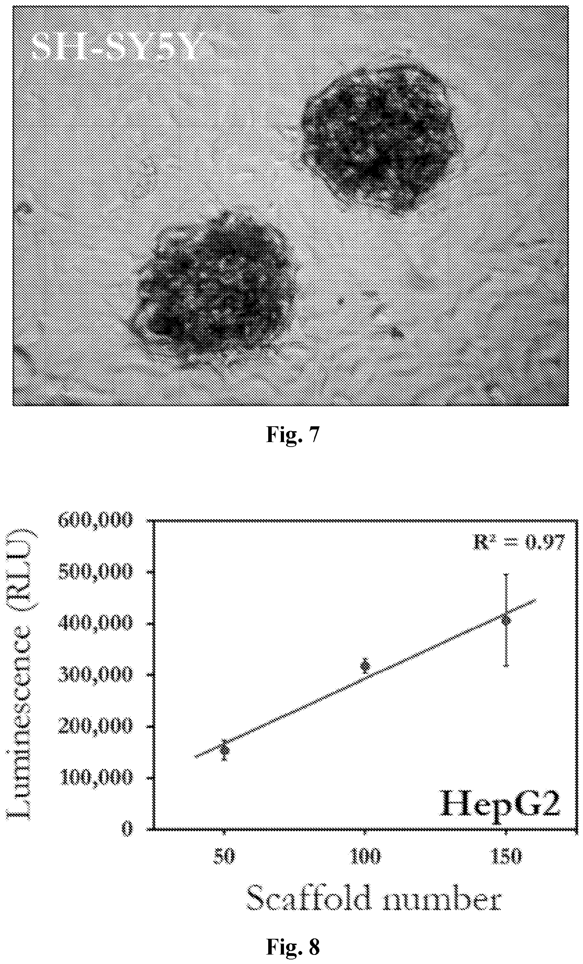

Fig. 7 is a light microscope image of SH-SY5Y neuroblastoma cells adhered to a number of microscaffolds of the invention after 4 days' growth.
Fig. 8 is a plot of the number of microscaffolds of the invention within the wells of a microwell plate, on the x axis, versus the luminescence generated by HepG2 cells (in relative light units, RLU) on the y axis, when the cells are attached to the microscaffolds. Briefly, cells on microscaffolds were incubated for a period of time (typically hours) with a non-luminescent substrate in the culture media which, when acted upon by enzymes within llve cells, converts the substance from non-luminscent to luminescent. The degree of luminescence is therefore proportional to the number and "health" of cells on scaffolds.

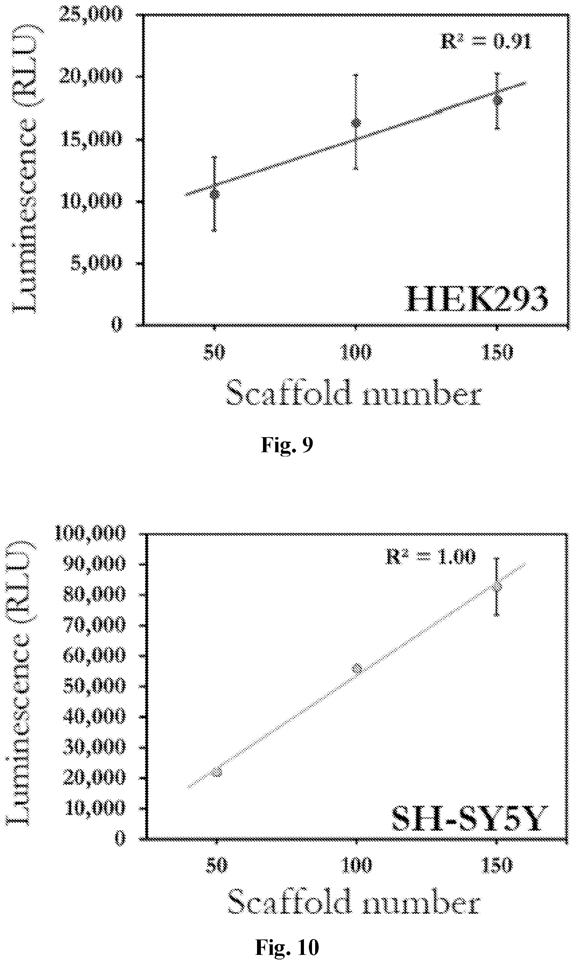

Fig. 9 is a plot of the number of microscaffolds of the invention within the wells of a microwell plate, on the x axis, versus the luminescence generated by HEK293 cells (in relative light units, RLU) on the y axis, when the cells are attached to the microscaffolds. Again, cells on microscaffolds were incubated for a period of time (typically hours) with a non-luminescent substrate in the culture media which, when acted upon by enzymes within llvecells, converts the substance from non-luminscent to luminescent. The degree of luminescence is therefore proportional to the number and "health" of cells on scaffolds.
Fig. 10 is a plot of the number of microscaffolds of the invention within the wells of a microwell plate, on the x axis, versus the luminescence generated by SH-SY5Y cells (in relative light units, RLU) on the y axis, when the cells are attached to the microscaffolds. Again, cells on microscaffolds were incubated for a period of time (typically hours) with a non-luminescent substrate in the culture media which, when acted upon by enzymes within llvecells, converts the substance from non-luminscent to luminescent. The degree of luminescence is therefore proportional to the number and "health" of cells on scaffolds.

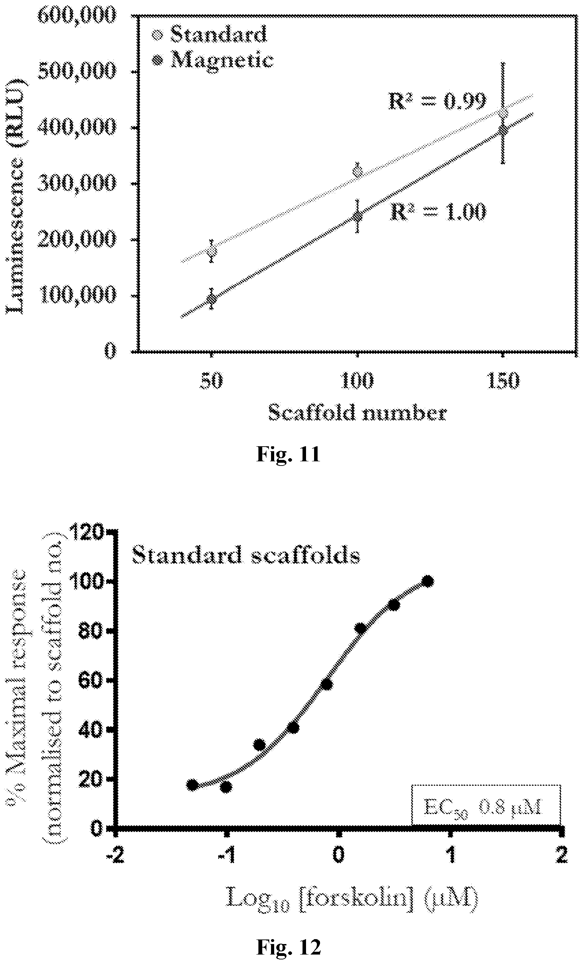

Fig. 11 is a plot of the number of microscaffolds within the wells of a microwell plate, on the x axis, versus the luminescence generated by a representative cell line of the above (in relative light units, RLU) on the y axis, when the cells are attached to standard microscaffolds of the invention (upper plot) or magnetic microscaffolds of the invention containing 1% by weight iron (II,III) oxide (lower plot). Briefly, cells on microscaffolds were incubated for a period of time (typically hours) with a non-luminescent substrate in the culture media which, when acted upon by enzymes within llvecells, converts the substance from non-luminscent to luminescent. The degree of luminescence is therefore proportional to the number and "health" of cells on scaffolds.
Fig. 12 is a plot of the % maximal response, to chemical stimulation by forskolin, of cells adhered to standard (non-magnetic) microscaffolds of the invention, normalised to the number of scaffolds (y axis), versus the base 10 logarithm of the concentration of forskolin in units of µM (x axis).

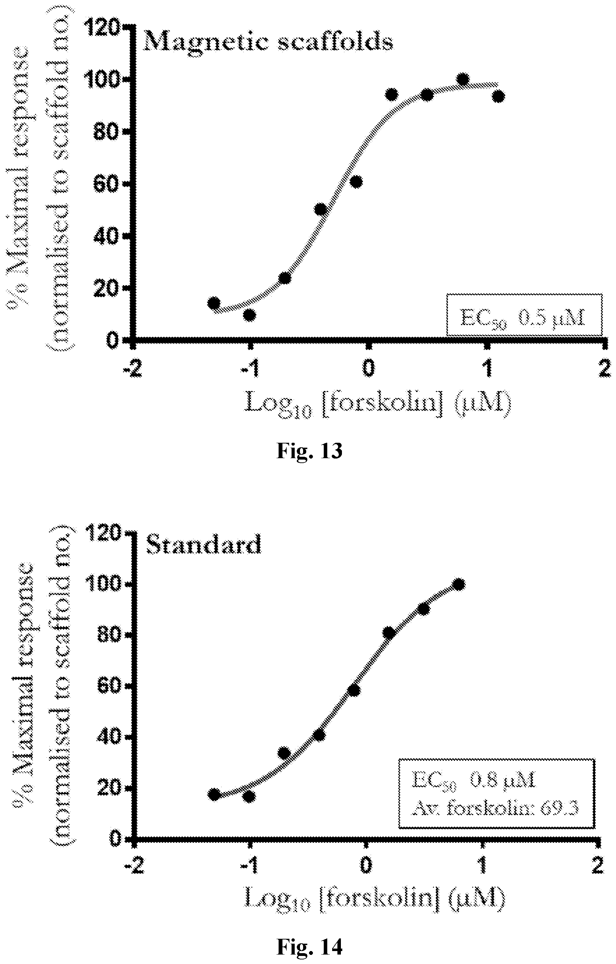

Fig. 13 is a plot of the % maximal response, to chemical stimulation by forskolin, of cells adhered to magnetic microscaffolds of the invention, normalised to the number of scaffolds (y axis), versus the base 10 logarithm of the concentration of forskolin in units of µM (x axis).
Fig. 14 is a plot of the % maximal response, to chemical stimulation by forskolin, of non-cryopreserved cells adhered to standard (non-magnetic) microscaffolds of the invention, normalised to the number of scaffolds (y axis), versus the base 10 logarithm of the concentration of forskolin in units of µM (x axis).

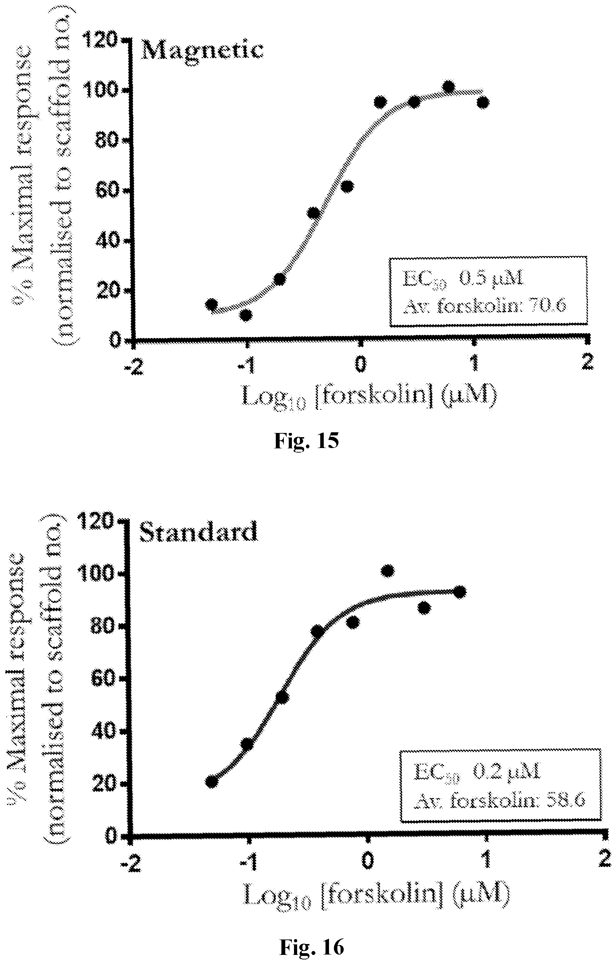

Fig. 15 is a plot of the % maximal response, to chemical stimulation by forskolin, of non-cry opreserved cells adhered to magnetic microscaffolds of the invention, normalised to the number of scaffolds (y axis), versus the base 10 logarithm of the concentration of forskolin in units of µM (x axis).
Fig. 16 is a plot of the % maximal response, to chemical stimulation by forskolin, of cells, adhered to standard (non-magnetic) microscaffolds of the invention, which had been cryopreserved on the scaffolds for 24 hours prior to the experiment, normalised to the number of scaffolds (y axis), versus the base 10 logarithm of the concentration of forskolin in units of µM (x axis).

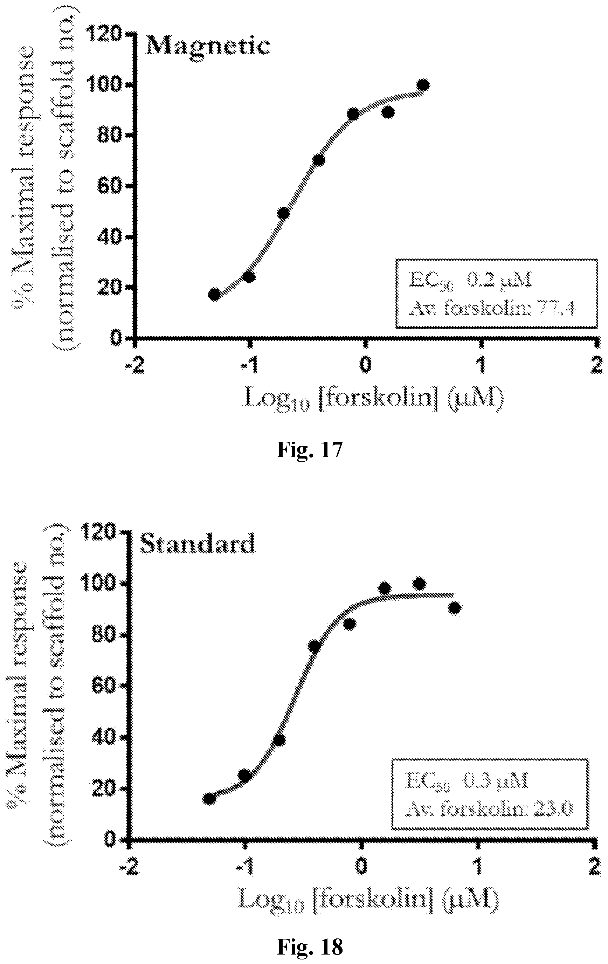

Fig. 17 is a plot of the % maximal response, to chemical stimulation by forskolin, of cells, adhered to magnetic microscaffolds of the invention, which had been cryopreserved on the scaffolds for 24 hours prior to the experiment, normalised to the number of scaffolds (y axis), versus the base 10 logarithm of the concentration of forskolin in units of µM (x axis).
Fig. 18 is a plot of the % maximal response, to chemical stimulation by forskolin, of cells, adhered to standard (non-magnetic) microscaffolds of the invention, which had been cryopreserved on the scaffolds for 48 hours prior to the experiment, normalised to the number of scaffolds (y axis), versus the base 10 logarithm of the concentration of forskolin in units of µM (x axis).

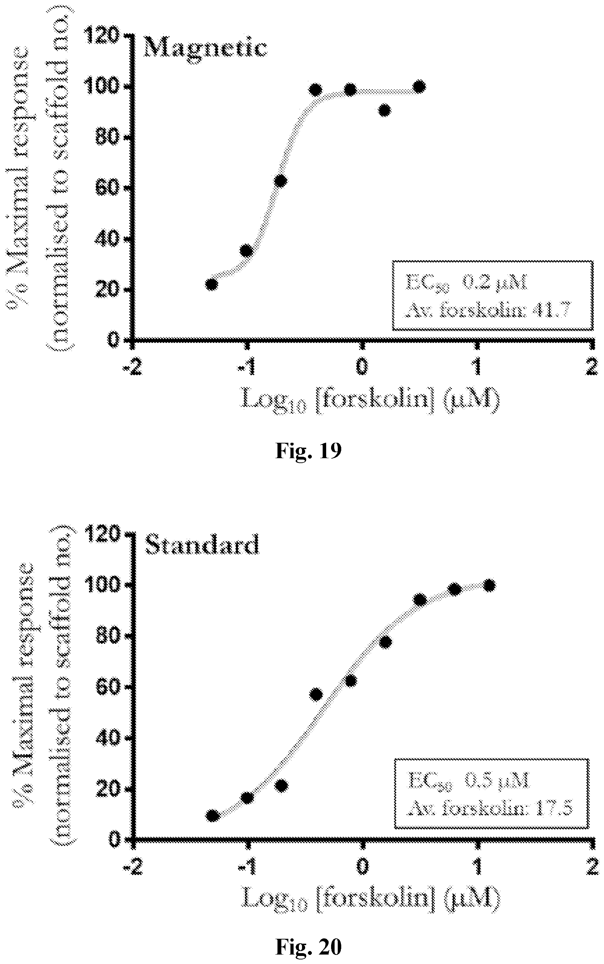

Fig. 19 is a plot of the % maximal response, to chemical stimulation by forskolin, of cells, adhered to magnetic microscaffolds of the invention, which had been cryopreserved on the scaffolds for 48 hours prior to the experiment, normalised to the number of scaffolds (y axis), versus the base 10 logarithm of the concentration of forskolin in units of µM (x axis).
Fig. 20 is a plot of the % maximal response, to chemical stimulation by forskolin, of cells, adhered to standard (non-magnetic) microscaffolds of the invention, which had been cryopreserved on the scaffolds for 72 hours prior to the experiment, normalised to the number of scaffolds (y axis), versus the base 10 logarithm of the concentration of forskolin in units of µM (x axis).

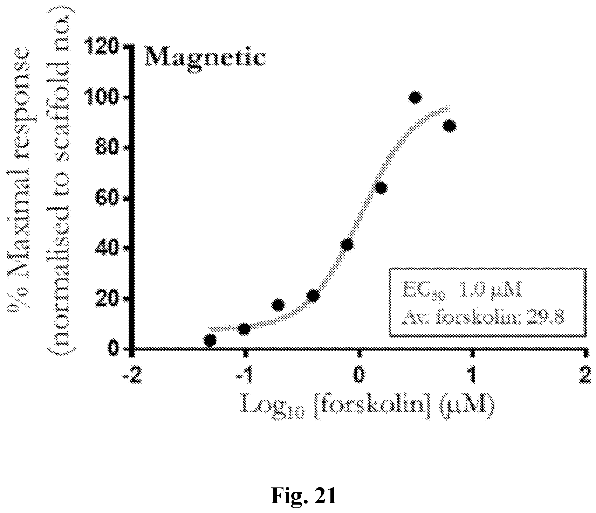

Fig. 21 is a plot of the % maximal response, to chemical stimulation by forskolin, of cells, adhered to magnetic microscaffolds of the invention, which had been cryopreserved on the scaffolds for 72 hours prior to the experiment, normalised to the number of scaffolds (y axis), versus the base 10 logarithm of the concentration of forskolin in units of µM (x axis).

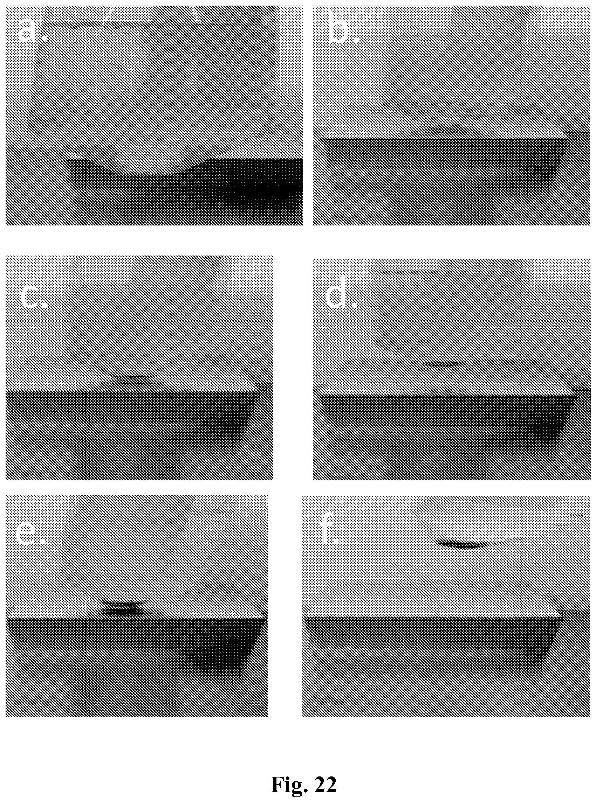

Fig. 22 shows freeze frames taken from a video which shows the ease of handling magnetic microscaffolds of the invention, and in particular the ease with which the magnetic microscaffolds can be suspended in an aqueous medium by constant stirring using an external magnetic stirring device. In particular, Figs. 22 (a) to (f) are timelapse photographic images of microscaffolds in solution within a 50ml conical tube showing, respectively: (a) microscaffolds randomly distributed in solution; (b) the microscaffolds are initially exposed to a neodymium magnet - note the dark coloured grains are microscaffolds being attracted to the magnet; (c) at 5 seconds of exposure - dark grains are attracted to the magnet; (d) at 10 seconds of exposure - a pool of dark grains begin to appear at the base of the tube; (e) within 15 seconds - almost all microscaffolds are attracted to the magnet at the bottom of the tube; and (f) that the tube can be removed with the microscaffolds remaining at the base of the tube.

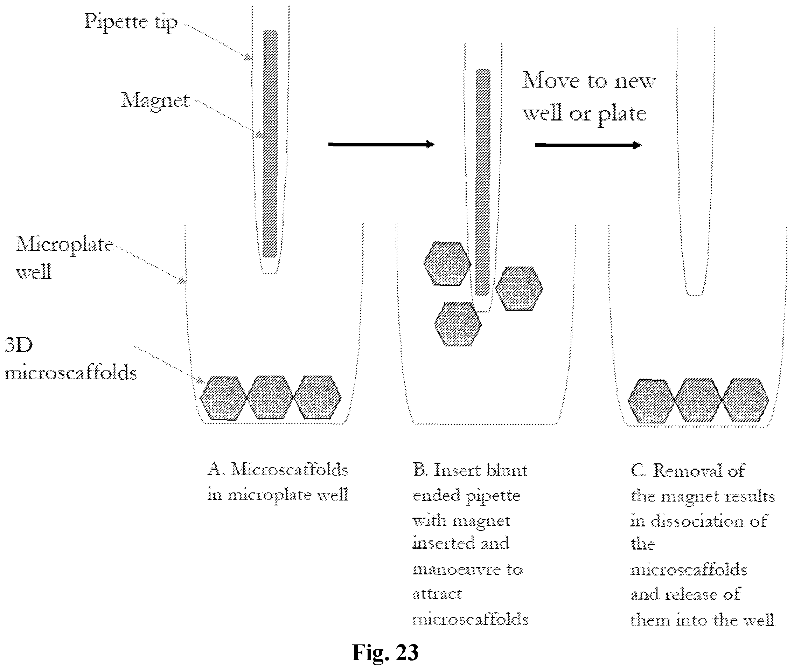

Fig. 23 is a schematic illustration showing how a magnet can be used to transfer the magnetic microscaffolds of the invention from one well to another well in one or more multi-well assay plates.

### DETAILED DESCRIPTION OF THE INVENTION

The invention provides a microscaffold comprising a porous particle, which particle: comprises a three dimensional network of electrospun fibres, which fibres comprise a biocompatible polymer; wherein the particle has the shape of a cylinder or a polygonal prism, wherein the diameter of the cylinder or polygonal prism is from 20 µm to 2000 µm, and wherein the height of the cylinder or polygonal prism is from 10 µm to 200 µm.

As used herein, the term "microscaffold" means a scaffold whose size is conveniently measured in micrometres (µm). Thus, the microscaffold of the invention has a particle size of less than or equal to 2000 µm.

The term "particle size" as used herein means the diameter of the particle if in an embodiment not according to the invention the particle is spherical or, if the particle is non-spherical, the volume-based particle size. The volume-based particle size is the diameter of a sphere whose volume is the same as the volume of the non-spherical particle in question.

Since the particle in this invention is porous, the term "volume", when used herein in connection with the particle, refers to the envelope volume of the particle, i.e. the sum of the volumes of both the solid in the particle and the voids within the particle, that is, within a close-fitting imaginary envelope that completely surrounds the particle.

A sphere having a diameter of 2000 µm has a volume of 4.19 × 10⁹ µm³, i.e. a volume of (4/3)π.r³ where r is 1000 µm. Accordingly, since the microscaffold of the invention comprises a porous particle which has a particle size of less than or equal to 2000 µm, the volume (i.e. envelope volume) of the porous particle is less than or equal to 4.19 × 10⁹ µm³.

Both the diameter of a spherical microscaffold particle not according to the invention and the volume of a non-spherical microscaffold particle of the invention can readily be measured by the skilled person, using known methods. Also, the diameter of a sphere having the same volume as the measured volume of a non-spherical microscaffold particle of the invention can readily be deduced. Thus, the skilled person is readily able to measure the particle size of a microscaffold of the invention.

The diameter of a spherical microscaffold particle not according to the invention may readily be measured by microscopy, i.e. by measuring the diameter of the spherical microscaffold as shown in a light microscope image of the microscaffold, or as shown in an electron micrograph of the microscaffold, for instance in an SEM image of the microscaffold. SEM images of microscaffolds of the invention are shown in Figures 3 and 4, although the microscaffolds shown in Figures 3 and 4 are non-spherical.

The volume of any microscaffold particle of the invention may be deduced by measuring the dimensions of the microscaffold needed to calculate its volume, and then calculating the volume. For example, if in an embodiment not according to the invention the particle is spherical, the diameter of the particle can be measured as discussed above, e.g. by measuring the diameter of the microscaffold as shown in an SEM image, and the volume of the particle can then be calculated using the mathematical formula for the volume of a sphere, which is equal to (4/3).π.r³, where r is the radius (i.e. half the diameter) of the sphere. If, on the other hand, the particle is non-sperical, for example if it has the shape of a cylinder or a polygonal prism, the volume can be obtained by measuring the height of the particle and the diameter or area of a face of the particle, and the volume of the particle can then be calculated using the mathematical formula for the volume of a cylinder or the volume of the relevant polygonal prism, as the case may be. For a a cylinder or a right polygonal prism this is the height or the cylinder or prism multiplied by the area of a face of the cylinder or prism, but often the area of the face may be deduced from the diameter of the face, hence the diameter of the face, rather than its area, often need only be measured.

For instance, if the particle has the shape of a cylinder, the volume can be obtained by measuring its height and also the diameter of one of its circular faces and then calculating the volume by using the mathematical formula for the volume of a cylinder, of π(d/2)².h, where d is the diameter and h is the height.

If on the other hand the particle has the shape of a hexagonal prism, and in particular a right, regular hexagonal prism, the volume can be obtained by measuring its height and also the diameter of one of its hexagonal faces (from vertex to opposite vertex) and then calculating the volume by using the formula for the volume of a right regular hexagonal prism, of [(3√3)/2].(d/2)².h where d is the diameter of a hexagonal face of the prism (from vertex to opposite vertex) and h is the height of the hexagonal prism.

The diameter of a hexagonal face of a hexagonal prism-shaped microscaffold (or indeed the diameter of the circular face of a cylindrical microscaffold, or the diameter of the face of a different polygonal prism-shaped microscaffold) can be measured by microscopy, i.e. by measuring the diameter of the face as shown in an SEM image of the microscaffold. See for example Figures 3 and 4, which show the hexagonal faces of hexagonal prism-shaped microscaffolds of the invention, whose diameters can be measured readily using the scales of the micrographs. The diameter (from vertex to opposite vertex) in these scaffolds is about 135 µm.

The height of a hexagonal prism-shaped microscaffold (or indeed of a cylindrical microscaffold, or a different polygonal prism-shaped microscaffold) can be measured using a micrometre. Typically, such microscaffolds are produced by laser-machining a scaffold layer, as described in Example 1 below, and the thickness of the scaffold layer from which the microscaffolds are produced can be measured using a micrometre. This thickness corresponds to the height of the microscaffolds produced. The thickness of the sheet of electrospun scaffold from which the hexagonal prism-shaped microscaffolds of Example 1 were produced was 50 µm. The heights of the resulting hexagonal prism-shaped microscaffolds were also therefore 50 µm.

The height of a hexagonal prism-shaped microscaffold can alternatively be measured by microscopy, i.e. by measuring the height of the microscaffold as shown in an electron miscroscope image (for instance in an SEM image) of the microscaffold.

As mentioned above the porous particle of the microscaffold of the invention has a particle size of less than or equal to 2000 µm. Usually, the porous particle has a particle size of less than 2000 µm. Often, for instance, the particle size is less than or equal to 1800 µm, or for instance less than or equal to 1500 µm. The particle size may for instance be less than or equal to 1300 µm, or for instance, less than or equal to 1200 µm, for example less than or equal to 1100 µm. The particle size may for instance be less than or equal to 1000 µm, or for instance, less than or equal to 900 µm, for example less than or equal to 700 µm.

The porous particle of the microscaffold of the invention may for instance has a particle size of less than or equal to 500 µm. It may, for example, have a particle size of less than or equal to 400 µm, or, for instance, less than or equal to 300 µm, such as less than or equal to 200 µm, or, for instance, less than or equal to 150 µm.

The porous particle of the microscaffold of the invention usually has a particle size of greater than 10 µm. Often, for instance the particle size is greater than or equal to 15 µm, for instance greater than or equal to 17 µm. The particle size may for instance be greater than or equal to 20 µm, or for instance, greater than or equal to 25 µm, for example greater than or equal to 30 µm. The particle size may for instance be greater than or equal to 35 µm, or for instance, greater than or equal to 40 µm, for example greater than or equal to 50 µm.

Often, the porous particle of the microscaffold of the invention has a particle size of greater than or equal to 60 µm. It may, for instance, have a particle size of greater than or equal to 70 µm, or, for example, greater than or equal to 80 µm, such as greater than or equal to 90 µm, or, for instance, greater than or equal to 100 µm.

Typically, therefore, the porous particle of the microscaffold of the invention has a particle size of from 10 µm to 2000 µm. Often, for instance the particle size is from 15 µm to 1800 µm, for instance from 17 µm to 1500 µm. The particle size may for example be from 20 µm to 1300 µm, or for instance, from 25 µm to 1200 µm, or for example from 30 µm to 1100 µm. The particle size may for instance be from 35 µm to 1000 µm, or for instance, from 40 µm to 900 µm, for example from 50 µm to 700 µm.

Often, the porous particle of the microscaffold of the invention has a particle size of from 60 µm to 500 µm. It may, for instance, have a particle size of from 70 µm to 400 µm, or, for example, from 80 µm to 300 µm, such as from 90 µm to 200 µm, or, for instance, from 100 µm to 150 µm.

The porous particle may take any shape but according to the invention is a cylinder or polygonal prism. It may for instance have an irregular shape. On the other hand, in an embodiment not according to the invention it may be spherical, or roughly. Alternatively, in an embodiment not according to the invention it may be spheroidal, for example in the shape of an oblate or prolate spheroid, or according to the invention it may be in the shape of a cylinder. However, the laser machining process by which porous particles of the invention may be produced lends itself to producing porous particles in the shape of polygonal prisms, for instance hexagonal prisms. Accordingly, often, the porous particle of the microscaffold of the invention has the shape of a polygonal prism. When the porous particle has the shape of a polygonal prism the polygonal prism is typically a right prism, i.e. it is typically a prism which has bases aligned one directly above the other and has lateral faces that are rectangular. Usually, the polygonal prism is a regular prism, i.e. a prism with bases that are regular polygons. More typically, the polygonal prism is a right regular prism, i.e. a right prism with bases that are regular polygons. Polygonal prisms are well known in the art; a polygonal prism may, for instance be a triangular prism, a tetragonal prism, a pentagonal prism, a hexagonal prism, a heptagonal prism, an octagonal prism, an enneagonal prism or a decagonal prism.

The porous particle may therefore have the shape of a polygonal prism. The polygonal prism may for instance be a triangular prism, a tetragonal prism, a pentagonal prism, a hexagonal prism, a heptagonal prism, an octagonal prism, an enneagonal prism or a decagonal prism. The polygonal prism may for instance be a hexagonal prism. Often, the polygonal prism is a right prism which may for instance be a right triangular prism, a right tetragonal prism, a right pentagonal prism, a right hexagonal prism, a right heptagonal prism, a right octagonal prism, a right enneagonal prism or a right decagonal prism. The polygonal prism may for instance be a right hexagonal prism. Often, the polygonal prism is a right regular prism, which may for instance be a right regular triangular prism, a right regular tetragonal prism, a right regular pentagonal prism, a right regular hexagonal prism, a right regular heptagonal prism, a right regular octagonal prism, a right regular enneagonal prism or a right regular decagonal prism. The polygonal prism may for instance be a right regular hexagonal prism. Figures 3 and 4 show microscaffolds of the invention which have the shapes of right regular hexagonal prisms.

The particle may have the shape of a cylinder or a polygonal prism. The polygonal prism may, for instance, be as further defined above, for instance it may be a right regular polygonal prism.

When the microscaffold particle has the shape of a cylinder or a polygonal prism, the height of the cylinder or polygonal prism (which typically corresponds to the thickness of the layer of scaffold material from which the microscaffolds are cut, when they are produced as described hereinbelow) may, for instance, be from 10 µm to 200 µm. Often, the height of the cylinder or polygonal prism is from 15 µm to 140 µm, or for instance from 20 µm to 120 µm, for example from 20 µm to 100 µm. The height of the cylinder or polygonal prism is often from 20 µm to 80 µm, for instance from 30 µm to 70 µm. It may for instance be from 35 µm to 65 µm, or for example from 40 µm to 60 µm. The height of the cylinder or polygonal prism is typically about 50 µm. The term "about 50 µm refers to 50 µm with a tolerance of ±10% (i.e. 50 µm ±5 µm).

When the microscaffold particle has the shape of a cylinder or a polygonal prism, the diameter of the cylinder or polygonal prism is typically from 20 µm to 2000 µm. The meaning of the diameter of a cylinder is well understood. The diameter of a polygon is also well understood, being the largest distance between any pair of vertices. Accordingly, the diameter of a polygonal prism is the largest distance between any pair of vertices on either of the polygonal faces of the prism. Thus, in the case of a hexagonal prism, and in particular in the case of a right regular hexagonal prism, the diameter is the diameter of either of the hexagonal faces of the prism, measured from a vertex (or intersection of two sides) of the hexagonal face, through the center of the face, to the opposite vertex of the face (where the two opposite sides of the face intersect).

The diameter of the cylinder or polygonal prism may for instance be from 20 µm to 1800 µm, or for instance from 25 µm to 1500 µm. Often, it is from 30 µm to 1200 µm, for instance from 30 µm to 1000 µm, or for instance from 35 µm to 800 µm. The diameter of the cylinder or polygonal prism may be from 35 µm to 500 µm, for instance from 40 µm to 400 µm. The diameter is often, for example, from 45 µm to 300 µm, or for instance from 50 µm to 200 µm, or from 60 µm to 180 µm. The diameter is typically, for instance, from 80 µm to 170 µm, for instance from 100 µm to 160 µm, for example from 110 µm to 150 µm.

In a preferred embodiment, the particle has the shape of a hexagonal prism. Typically, it has the shape of a right, regular hexagonal prism. The particle size of the particle which has the shape of a hexagonal prism may be as defined above for the microscaffold of the invention more generally. Typically, therefore, it has a particle size of from 10 µm to 2000 µm. The height and diameter of the hexagonal prism may be as defined above for the height and diameter of the polygonal prism. For instance, the height of the hexagonal prism may be from 10 µm to 200 µm and the diameter may be from 20 µm to 2000 µm. A microscaffold particle having the shape of a right, regular hexagonal prism with a height of 200 µm and a diameter of 2000 µm has a volume of 5.20 × 10⁸ µm³, and therefore a volume-based particle size of 997 µm (which is the diameter of a sphere having the same volume). A microscaffold particle having the shape of a right, regular hexagonal prism with a height of 10 µm and a diameter of 20 µm, on the other hand, has a volume of 2598 µm³ and a volume-based particle size of 17 µm. However, the height and/or diameter of the hexagonal prism may be as further defined above for the polygonal prism. Thus, the height of the hexagonal prism may, for instance be from 15 µm to 140 µm, and the diameter may be from 20 µm to 1800 µm. The height may, for example be from 20 µm to 120 µm, and the diameter may be from 25 µm to 1500 µm. Often, for instance, the height of the hexagonal prism is from 20 µm to 100 µm, for instance from 20 µm to 80 µm, and the diameter is from 30 µm to 1200 µm, or for instance from 30 µm to 1000 µm. The height of the hexagonal prism is typically from 30 µm to 70 µm, and the diameter is from 35 µm to 800 µm or for instance from 35 µm to 500 µm. The height may for example be from 35 µm to 65 µm, and the diameter of the hexagonal prism may be from 40 µm to 400 µm, for instance from 45 µm to 300 µm, or for instance from 50 µm to 200 µm, or from 60 µm to 180 µm. Thus, the height of the hexagonal prism may for instance be from 20 µm to 80 µm, and the diameter of the hexagonal prism may be from 40 µm to 400 µm. Usually, the height of the hexagonal prism is about 50 µm, and the diameter of the hexagonal prism is from 80 µm to 170 µm, for instance from 100 µm to 160 µm, or, for example, from 110 µm to 150 µm. The term "about 50 µm refers to 50 µm with a tolerance of ±10% (i.e. 50 µm ±5 µm).

Advantageously, microscaffolds within the particle size, height and diameter ranges described herein are generally large enough to grow cell cultures which extend in all three dimensions enough to provide the benefits of a 3D cell culture versus a 2D layer of cells. As discussed hereinbefore, such benefits include more accurately mimicking *in vivo* conditions and thereby reducing false positives early on in the drug screening process. At the same time, cell cultures generated within such microscaffolds are thin enough to render the culture easy to image using high throughput screening methods. Thicker cell cultures, for instance, can be time consuming to analyse by microscopy due to the need to collect more images in the z direction (i.e. a higher degree of "z-stacking" is necessary to image the sample) and such methods may therefore be less suitable for high throughput screening. Also, microscaffolds within these particle size, height and diameter ranges are generally small enough to manufacture very quickly and inexpensively using techniques such as electrospinning, and thin enough to facilitate rapid growth of 3D cell cultures. It can be time consuming and expensive to grow and then maintain 3D cell cultures in thicker scaffolds.

The porous particle of the microscaffold comprises a three dimensional network of fibres. The fibres are typically nanofibres. As used herein, the term "nanofibre" means a microscopic fibre whose diameter is conveniently measured in nanometres (nm) or micrometres (µm). The mean diameter of the fibres used in the present invention is typically from 500 nm to 10 µm.

The use of a porous particle, comprising a three-dimensional network of fibres as the microscaffold material provides certain advantages. Batch to batch reproducibility is one such advantage, and compatibility with current automation equipment is another. Networks of fibres with specific mean fibre diameters and low standard deviations from the mean can be produced very consistently, for instance by electrospinning or by other suitable methods for producing non-woven fibrous networks, such as melt spinning, dry spinning, wet spinning and extrusion. Electrospinning has been found particularly suitable for consistent reproduction of nanofibre networks with desired mean fibre diameters and low standard deviations from the mean.

The present invention therefore provides for a high degree of control over the mean microscaffold fibre diameter, and over the standard deviation from the mean, and microscaffolds in which fibres have specific mean diameters with low variance from the mean can therefore be produced. This in turn provides control over the microscaffold porosity and pore size, which, together with fibre diameter, are important factors that affect whether or not 3D cell cultures can successfully grow in the microscaffold. As the skilled person will appreciate, a scaffold should be sufficiently porous, with large enough pores, to allow cells to infiltrate the scaffold and grow in 3D culture. On the other hand, the pore size cannot be too large as cells will then effectively fall through the pores and not fill the entire volume of the material; the material will not then be an effective scaffold. The porosity, average pore diameter and the average fibre diameter of a non-woven network are interrelated as explained, for instance in Greiner and Wendorff, Angew. Chem. Int. Ed. 2007, 46, 5670-5703. Thus, the present invention provides for a particularly high level of control over microscaffold fibre diameter, porosity and pore size in order to facilitate the growth of a variety of cell types in 3D culture. Alternative technologies for 3D cell growth, on the other hand, including for instance hydrogel scaffolds, do not allow for such a high degree of control over such factors. It is particularly difficult to manufacture suitable hydrogel materials with desired porosities and pore sizes consistently from batch to batch. Batch to batch reproducibility is therefore problematic for such materials. Hydrogel scaffold materials are also incompatibile with current automation equipment.

Usually, the fibres in the porous three-dimensional network of the microscaffold are randomly oriented. In another embodiment, however, the fibres in the microscaffold particle are aligned. An electrospinning process for aligned fibre production is for instance described in WO2011/011575, and such processes are also described in Z.-M. Huang et al., Composites Science and Technology 63 (2003) 2223-2253 and in Greiner and Wendorff, Angew. Chem. Int. Ed. 2007, 46, 5670-5703.

As mentioned above, the porous network of fibres employed in the microscaffold can be produced by electrospinning, as detailed further below, or by other suitable methods which are known to the skilled person including, but not limited to, melt spinning, dry spinning, wet spinning and extrusion. Electrospinning is preferred. Thus the fibres in the network may comprise electrospun, melt-spun, dry-spun, wet-spun or extruded nanofibres. Usually, however, the fibres comprise electrospun nanofibres.

The fibres are typically therefore electrospun fibres.

Usually, the mean diameter of the fibres in the microscaffold particle is from 500 nm to 10 µm. More typically, the mean diameter of the fibres is from 800 nm to 8 µm, or for instance from 1 µm to 7 µm. Often, for instance, the mean diameter of the fibres is from 2 µm to 6 µm.

In a preferred embodiment, the mean diameter of the fibres is from 2 µm to 6 µm. For instance, the mean diameter of the fibres may be from 2.5 µm to 5.5 µm, or for instance from 3 µm to 5 µm. For instance, the mean diameter of the polymer nanofibres may be from 3.5 µm to 4.5 µm. Usually, in this embodiment the mean diameter of the polymer nanofibres is from 3.8 µm to 4.2 µm, for instance about 4.0 µm.

Typically, the relative standard deviation from said mean is less than or equal to 25%. Preferably, the relative standard deviation from said mean is less than or equal to 20%. For instance, the relative standard deviation from said mean may be less than or equal to 15%.

In a preferred embodiment, the mean diameter of the fibres is from 2 µm to 6 µm, and the relative standard deviation from said mean is less than or equal to 25%. However, the relative standard deviation from said mean in this embodiment may be less than or equal to 20%, or for instance less than or equal to 15%.

The mean diameter of the fibres may for instance be from 2.5 µm to 5.5 µm, and the relative standard deviation from said mean may be less than or equal to 25%. The relative standard deviation from said mean in this embodiment may however be less than or equal to 20%, or for instance less than or equal to 15%.

The mean diameter of the polymer nanofibres may for instance be from 3 µm to 5 µm, for instance about 4 µm, and the relative standard deviation from said mean may less than or equal to 25%. However, the relative standard deviation from said mean may be less than or equal to 20%, or for instance less than or equal to 15%.

Typically, the mean diameter of the fibres in the scaffold is measured by Scanning Electron Microscopy (SEM). Usually, the relative standard deviation from said mean is also measured by SEM. Automated image characterisation is generally performed using a Phenom Fibremetric SEM system (Phenom World), which enables the automated analysis of multiple images in order to determine the mean fibre diameter and the relative standard deviation. The Fibremetric software automatically identifies the location of the fibres due to the contrast within the captured SEM image and measures the diameter of each fibre 20 times at a specific location. Typically around 100 of such measurements are performed per image. Alternatively, the diameter of the fibres can be obtained via manual measurements / analysis of multiple SEM images.

Fibres with mean diameters in these ranges and with these standard deviations from the mean provide porous networks which facilitate the growth of three-dimensional (3D) cell cultures with a high degree of consistency and reproducibility not only from microscaffold to microscaffold within a batch, but also from batch to batch, for a variety of cell types.

The mean pore size in the microscaffold is typically from 10 µmto 20 µm.Pore size can be difficult to measure accurately, though, as pore size depends on how far through the scaffold you measure and no two pores are the same shape due to the random orientation of the nanofibres. The pore size is tuned roughly to match a typical cell diameter (approx. 20 microns) however the loose nature of the nanofibres allows for cells to migrate into the microscaffold by pushing the nanofibres aside. With respect to total porosity, the microscaffolds typically have a porosity of greater than 75%. In other words, the microscaffolds are typically greater than 75% air, by volume. The microscaffolds may for instance have a porosity of greater than 80%. Typically, the porosity is from about 75% to about 85%. In some embodiments, however, the microscaffolds may have a porosity of greater than 90%, for instance from about 90% to about 95%.

The fibres in the microscaffold particle comprise a polymer. Any biocompatible polymer can be employed, and the polymer may for instance be a natural polymer or a synthetic polymer. In some embodiments, the polymer is a bioerodable polymer.

Preferably the polymer is one which has little or no autofluorescence. In particular, it is preferred that the polymer is one which has little or no autofluorescence in the wavelengths typically used for fluorescent assays, for instance at wavelengths in the visible region of the electromagnetic spectrum. The nanofibres in the scaffold layer typically therefore comprise a polymer which has little or no autofluorescence at wavelengths of from 390 nm to 710 nm.

The fibres in the microscaffold may for instance comprise any of the following polymers:
poly(lactide); poly(glycolic acid); poly(ε-caprolactone); polyhydroxybutyrate; polystyrene; polyethylene; polypropylene; poly(ethylene oxide); a poly(ester urethane); poly(vinyl alcohol); polyacrylonitrile; polylactide; polyglycolide; polyurethane; polycarbonate; polyimide; polyamide; aliphatic polyamide; aromatic polyamide; polybenzimidazole; poly(ethylene terephthalate); poly[ethylene-co-(vinyl acetate)]; poly(vinyl chloride); poly(methyl methacrylate); poly(vinyl butyral); poly(vinylidene fluoride); poly(vinylidene fluoride-co-hexafluoropropylene); cellulose acetate; poly(vinyl acetate); poly(acrylic acid); poly(methacrylic acid); polyacrylamide; polyvinylpyrrolidone; poly(phenylene sulfide); hydroxypropylcellulose; polyvinylidene chloride, polytetrafluoroethylene, a polyacrylate, a polymethacrylate, a polyester, a polysulfone, a polyolefin, polysilsesquioxane, silicone, epoxy, cyanate ester, a bis-maleimide polymer; polyketone, polyether, polyamine, polyphosphazene, polysulfide, an organic/inorganic hybrid polymer or a copolymer thereof, for instance, poly(lactide-co-glycolide); polylactide-co-poly(ε-caprolactone) or poly(L-lactide)-co-poly(ε-caprolactone); or a blend thereof, for instance a blend of poly(vinyl alcohol) and poly(acrylic acid).

The fibres in the microscaffold may comprise a bioerodible polymer, for instance a bioerodible polymer selected from poly(L-lactide); poly(glycolic acid); polyhydroxybutyrate; and poly(ester urethanes).

The fibres in the microscaffold may alternatively for instance comprise a biopolymer, or a blend of a biopolymer with a synthetic polymer. The following biopolymers and blends of biopolymers with synthetic polymers may for instance be used:
collagen; collagen/poly(ethylene oxide); collagen/poly(ε-caprolactone); collagen/polylactide-co-poly(ε-caprolactone); gelatin; gelatin/poly(ε-caprolactone); gelatin/ poly(ethylene oxide); casein/poly(vinyl alcohol); casein/poly(ethylene oxide); lipase; cellulase/poly(vinyl alcohol); bovine serum albumin/poly(vinyl alcohol); luciferase/poly(vinyl alcohol); α-chymotrypsin; fibrinogen; silk; regenerated silk; regenerated *Bombyx mori* silk; Bombyx mori silk/poly(ethylene oxide); silk fibroin; silk fibroin/chitosan; silk fibroin/chitin; silk/poly(ethylene oxide) (coaxial); artificial spider silk; chitin; chitosan; chitosan/poly(ethylene oxide); chitosan/poly(vinyl alcohol); quaternized chitosan/poly(vinyl alcohol); hexanoylchitosan/polylactide; cellulose; or cellulose acetate.

The fibres in the microscaffold may alternatively for instance comprise a blend of two or more polymers, a copolymer (which may for instance be a block copolymer), or a blend of a polymer with an inorganic material.

Non-limiting examples of such blends of two or more polymers include a polyvinylpyrrolidone/polylactide blend; a polyaniline/polystyrene blend; a polyaniline/poly(ethylene oxide) blend; a poly(vinyl chloride)/polyurethane blend, a poly[(m-phenylene vinylene)-co-(2,5-dioctyloxy-p-phenylene vinylene)]/poly(ethylene oxide) blend; a poly[2-methoxy-5-(2'-ethylhexyloxy)-1,4-phenylene vinylene] (MEH-PPV)/polystyrene blend, a polyaniline/polystyrene blend; a polyaniline/polycarbonate blend, a poly(ethylene terephthalate)/ poly(ethylene terephthalate)-co-poly(ethylene isophthalate) blend, a polysulfone/polyurethane blend; a chitosan/polylactide blend, a polyglycolide/chitin blend, and a polylactide/poly(lactide-co-glycolide) blend.

Non-limiting examples of such block copolymers systems include polylactide-b-poly(ethylene oxide) block copolymers; poly(lactide-co-glycolide)-b-poly(ethylene oxide) block copolymers; poly[(trimethylene carbonate)-b-(s-caprolactone)] block copolymers; polystyrene-b-polydimethylsiloxane and polystyrene-b-polypropylene block copolymers; polystyrene-b-polybutadiene-b-polystyrene block copolymers and polystyrene-b-polyisoprene block copolymers.

Non-limiting examples of blends of a polymer with an inorganic material from which fibres can be produced include: montmorillonite with polyamide 6, polyamide 6,6 and poly(vinyl alcohol), poly(methyl methacrylate), or polyurethane as the carrier material; a blend of a polymer carrier and noble metal nanoparticles, for instance poly(acrylonitrile)-co-poly(acrylic acid)/Pd; poly(ethylene oxide)/Au; polyvinylpyrrolidone/Ag; and poly(acrylonitrile)/Ag; a blend of a polymer carrier and magnetic nanoparticles, for instance poly(ethylene oxide) (or poly(vinyl alcohol))/Fe₃O₄, poly(ε-caprolactone)/FePt; polyurethane/MnZnNi; and poly(methyl methacrylate)/Co; a blend of a polymer and carbon nanotubes, for instance carbon nanotubes blended with poly(acrylonitrile), poly(ethylene oxide), poly(vinyl alcohol), polylactide, polycarbonate, polystyrene, polyurethane, or poly(methyl methacrylate); a blend of a polymer and a metal oxide or metal sulfide, for instance: polymer/TiO₂ wherein the polymer may for instance be with polyvinylpyrrolidone, poly(vinyl acetate), or poly(acrylonitrile); polymer/ZrO₂ wherein the polymer may for instance be polyvinylpyrrolidone, poly(vinyl acetate) and poly(vinyl alcohol); and blends of a polymer or polymers with: ZnO, CuO, NiO, CeO₂, Mn₃O₄, Mn₂O₃/Mn₃O₄, MoO₃, BaTiO₃, Y₂O₃, Gd₂O₃, Ta₂O₅, Co₃O₄, Ba_{0.6}Sr_{0.4}TiO₃, SiO₂, CdS, PbS, Ag₂S, iron(II) oxide (FeO), iron(II,III) oxide (Fe₃O₄), or iron(III) oxide (Fe₂O₃), or another magnetic material which may be as further defined herein below.

Thus, the fibres in the microscaffold may for instance comprise any of the materials listed in the preceding paragraphs. Scaffolds of nanofibers comprising the above polymers, copolymers, blends two or more polymers, and blends of a polymer with an inorganic material, can be produced by electrospinning, as detailed in in Greiner and Wendorff, Angew. Chem. Int. Ed. 2007, 46, 5670-5703.

In a preferred embodiment, the fibres in the microscaffold comprise poly(L-lactide). It is a finding of the invention that microscaffolds comprising a porous particle comprising a three-dimensional network of poly(L-lactide) fibres are particularly useful, owing to the advantageous handling properties of the scaffold material, the biocompatibility and biodegradability of the polymer, the low autofluorescence of the polymer, and the ability of such microscaffolds to support 3D cell culture growth for a variety of cell types. Sheets of scaffold material comprising poly(L-lactide) nanofibres handle particularly well, allowing for ease of manufacture of the microscaffolds of the invention. The material was found to handle like a sheet of paper, i.e. it is flexible but not brittle or weak, and also not stretchy or elastic. Poly(L-lactide) is also biocompatible and biodegradable, and is therefore a suitable polymer for use in microscaffolds for growing three-dimensional cell cultures. A further advantage of poly(L-lactide) is that the microscaffolds which comprise poly(L-lactide) of the present invention have little or no autofluorescence in the wavelengths typically used for fluorescent assays.

Usually, therefore, the fibres of the porous particle of the microscaffold of the invention comprise poly(L-lactide). The fibres are typically nanofibres. The fibres in the microscaffold may comprise a further polymer, or an inorganic material (such as a magnetic material), in addition to the poly(L-lactide) polymer. Such further polymers and inorganic materials can be selected from those listed herein. In some embodiments, however, the nanofibres in the microscaffold of the invention contain only poly(L-lactide), or at least substantially only poly(L-lactide). Thus, in some embodiments, the fibres in the microscaffold consist essentially of poly(L-lactide). For instance, the fibres may consist of poly(L-lactide).

Typically, the poly(L-lactide) is poly(L-lactide) which has an inherent viscosity midpoint of from 1.0 dl/g to 2.5 dl/g. The poly(L-lactide) may for instance have an inherent viscosity midpoint of from 1.5 dl/g to 2.1 dl/g, for instance an inherent viscosity midpoint of about 1.8 dl/g.

The poly(L-lactide) typically has a weight average molecular weight (M_{w}) of from 50,000 g/mol to 400,000 g/mol. Preferably, the poly(L-lactide) has a M_{w} of from 180,000 g/mol to 260,000 g/mol, for instance about 220 kg/mol, such as 221 kg/mol.

It is a further finding of the invention not only that magnetic material may be incorporated into the microscaffold, but also that magnetic material can be incorporated into the microscaffold without adversely affecting the viability of cells grown in the microscaffold. The inventors have also found that the presence of the magnetic material does not adversely affect the ability of the cell cultures to respond pharmacologically to chemical stimuli, or the ability of the cell-laden microscaffolds to be cryopreserved. The presence of a magnetic material in the microscaffold provides certain advantages. It increases the ease with which the microscaffolds may be manipulated. Indeed, magnetic microscaffolds may be manipulated using one or more external magnets, allowing for magnetic transfer of cell-laden scaffolds from one location to another location, or indeed for retaining the microscaffolds in a particular position whilst the cell medium is renewed or replenished. The presence of a magnetic material also increases the ease with which the microscaffolds may be maintained in suspension, for instance by using an externally-situated magnetic stirring device.

Accordingly, the porous particle of the microscaffold may further comprise a magnetic material. The magnetic material may be any magnetic material which is attracted to a magnet. The magnetic material may comprise nanoparticles of the magnetic material. The nanoparticles may have a particle size of from 10 to 500 nm, for instance from 50 to 100 nm. The magnetic material may be a paramagnetic, ferromagnetic, ferrimagnetic or superparamagnetic material. However, since paramagnetic materials are typically only weakly attracted to a magnet, the magnetic material is preferably a ferromagnetic, ferrimagnetic or superparamagnetic material. The magnetic material may for instance comprise iron; iron oxide; nickel; cobalt; an alloy comprising iron, nickel and/or cobalt; a rare earth magnet (e.g. a ferromagnetic compound or alloy comprising a lanthanide element); a ferrite; a magnetic mineral (e.g. magnetite or lodestone); or a superparamagnetic material. Superparamagnetic materials include superparamagnetic nanoparticles, for instance nanoparticles of a ferromagnetic or ferrimagnetic material. The nanoparticles may have a particle size of from 10 to 500 nm, for instance from 50 to 100 nm. The ferromagnetic or ferrimagnetic material may be any of those mentioned above, but it often comprises an oxide of iron.

Usually, the magnetic material comprises an oxide of iron, for instance iron (II,III) oxide. Often, the magnetic material comprises iron oxide nanoparticles. For instance, the magnetic material may comprise iron oxide nanoparticles having a particle size of from 30 to 300 nm, or for instance from 50 to 100 nm. The iron oxide may comprise iron (II,III) oxide.

The magnetic material is typically incorporated into the polymer fibres themselves. If the fibres are produced by electrospinning then the magnetic material may be incorporated into the fibres by including it in the solution that is used in the electrospinning process. If the fibres are produced by another method for producing non-woven fibrous networks, such as for example melt spinning, dry spinning, wet spinning and extrusion, the magnetic material may be incorporated into the fibres by including it in the relevant starting material, e.g. the starting material that is spun or extruded.

Alternatively, the magnetic material may be incorporated into or onto the polymer fibres after the fibres are produced, for instance by grafting or bonding the magnetic material to the fibres by a further chemical reaction after the fibres are produced, or for instance by physically coating or adsorbing the material onto, or impregnating it into, the fibres after production.

Accordingly, the fibres of the microscaffold which comprise the polymer, typically further comprise said magnetic material.

The concentration of the magnetic material in the microscaffold of the invention may, for instance, be less than or equal to 5.0 weight %, based on the total weight of the microscaffold, for instance from 0.01 weight % to 5.0 weight %. Often, the concentration of the magnetic material in the microscaffold is less than or equal to 3.0 weight %, based on the total weight of the microscaffold, and more typically less than or equal to 2.0 weight %. It may for instance be from 0.05 weight % to 2.0 weight %, based on the total weight of the microscaffold, or for instance from 0.1 weight % to 1.7 weight %, from 0.5 weight % to 1.5 weight %, or for example from 0.7 weight % to 1.3 weight %. The concentration of the magnetic material in the microscaffold may for instance be about 1 weight %.

In addition to, or as an alternative to, the magnetic material, the fibres may further comprise one or more useful biological or biocompatible materials. The fibres may, for instance, further comprise one or more of the following useful materials:
- an extracellular matrix molecule
- a peptide
- a nucleic acid
- a fluorescent dye
- streptavidin
- biotin

Extracellular matrix molecules which the fibres may usefully include are, for example, hyaluronidase, poly-D-lysine, laminin, vitronectin, fibronectin and collagen. A nucleic acid molecule that could usefully be employed is clonal DNA.

Again, if the fibres are produced by electrospinning then the useful materials above may be incorporated into the fibres by including it in the solution that is used in the electrospinning process. If the fibres are produced by another method for producing non-woven fibrous networks, such as for example melt spinning, dry spinning, wet spinning and extrusion, the magnetic material may be incorporated into the fibres by including it in the relevant starting material, e.g. the starting material that is spun or extruded. Alternatively, the one or more useful biological or biocompatible materials may be attached to the fibres after the fibres are produced. The one or more useful biological or biocompatible materials may for instance be grafted or bonded to the fibres by a further chemical reaction after the fibres are produced, or for instance such materials may be physically coated or adsorbed onto, or impregnated into, the fibres after production.

The microscaffold of the invention is capable of supporting 3D culture growth for a wide variety of cell types, including cell lines, stem cells and primary cells.

Accordingly, the microscaffold of the invention may further comprise cells attached to the microscaffold. The cells may for instance be a cell line, stem cells or primary cells. The cells are typically mammalian cells. The mammalian cells may for instance be a cell line, stem cells or primary cells. Typically, the microscaffold further comprises extracellular matrix.

The microscaffold of the invention may further comprise a cell culture within at least a portion of the microscaffold. The cell culture comprises biological cells, and typically also extracellular matrix. The cells are typically mammalian cells, and may for instance be a cell line, stem cells or primary cells. The mammalian cells may for instance be liver cells (hepatocytes), kidney cells or nerve cells, for instance neuroblastoma cells. The cell culture is typically a three-dimensional (3D) cell culture.

The microscaffold of the invention may be produced by the process of the invention, which comprises:
(a) producing a scaffold layer, which scaffold layer comprises a porous, three dimensional network of fibres, which fibres comprise a polymer; and
(b) cutting the scaffold layer to produce a porous particle, which particle comprises said three dimensional network of fibres and has a particle size of less than or equal to 2000 µm.

The scaffold layer, which comprises said porous three-dimensional network of fibres, can be produced by electrospinning or by another suitable method for producing a non-woven fibrous network, such as melt spinning, dry spinning, wet spinning and extrusion. Electrospinning is particularly suitable for consistent reproduction of scaffold layers, for use in producing microscaffolds, which have specific desired mean fibre diameters and low standard deviations from the mean. Thus, electrospinning provides for high batch-to-batch reproducibility. It also allows the properties of the fibrous networks (in particular the fibre diameter, and therefore porosity and pore size) to be tuned to suit the growth of particular cell types, and for such tailored scaffolds to be produced consistently with high batch-to-batch reproducibility.

The process of electrospinning per se is well known, and is described for instance in the following review articles: Z.-M. Huang et al., Composites Science and Technology 63 (2003) 2223-2253 and in Greiner and Wendorff, Angew. Chem. Int. Ed. 2007, 46, 5670-5703.

Typically, a polymer or polymer blend from which a fibrous network is to be produced is dissolved in an appropriate solvent until a homogeneous solution of the required concentration is obtained. The concentration of the polymer solution must generally be high enough to achieve adequate chain entanglements in order for a continuous fibre to be formed. The polymer solution is typically then loaded into a vessel, usually a syringe, connected to a conductive (typically metal) capillary. The capillary is connected to a high voltage (usually to the positive terminal of a high voltage DC power supply), at a fixed distance from an earthed collection device. The collection device may be metallic, and is typically covered in a collection substrate onto which the fibres are deposited. The collection device is preferably rotatable, to ensure uniform deposition of the material. Fibres are typically produced by passing the polymer solution at a fixed flow rate through the metal capillary whilst applying a high voltage to the capillary in order to establish an electric field between the capillary and the collection device. The applied voltage should be high enough to overcome the surface tension of the polymer droplet at the tip of the capillary. As the charge builds at the surface of the droplet, the surface area has to increase to accommodate the additional charge, this occurs through the formation of a Taylor Cone from the droplet, from which a continuous fibre is extracted. As the fibre travels towards the grounded collector, the solvent rapidly evaporates and the fibre is further elongated due to instabilities arising from the columbic repulsions of the surface charges on the jet. The instabilities in the jet resulting from the high charge density cause the jet to whip about rapidly resulting in a nano/micro diameter solid (dry) filament. The collector is rotated slowly (at a rate of around 100 rpm) resulting in the deposition of a non-woven fibrous membrane on the substrate. After a fixed amount of material has been deposited to generate a layer or membrane of a particular desired thickness, the layer or membrane is dried in order to remove any residual solvent/moisture from the fibres. Typically, it is dried under vacuum, for instance for 24-48 hours at room temperature (approx. 25°C).

Typically, therefore, the process of the invention for producing a microscaffold of the invention further comprises: producing the scaffold layer by electrospinning a nanofibre precursor solution onto a collection substrate, wherein the nanofibre precursor solution comprises said polymer dissolved in a solvent.

Usually, said electrospinning comprises:
providing a fibre forming module adjacent a fibre collection device and spaced therefrom, wherein the fibre forming module comprises a dispensing capillary and wherein the fibre collection device is earthed and comprises a collection substrate;
applying a voltage across the dispensing capillary and the fibre collection device; and,
whilst applying said voltage,
feeding (preferably pumping) said nanofibre precursor solution through the dispensing capillary, thereby causing deposition of said scaffold layer on the collection substrate.

Any suitable polymer may be employed in the nanofibre precursor solution used in the electrospinning process. The polymer employed may be any of the polymers listed above in relation to the microscaffold of the invention, or any of the copolymers, blends of two or more polymers, and blends of a polymer with an inorganic material listed hereinbefore. All of those polymers, copolymers and blends can be used in an electrospinning process to produce a porous three dimensional network of nanofibres, as detailed in Greiner and Wendorff, Angew. Chem. Int. Ed. 2007, 46, 5670-5703. The polymer used is generally of course a biocompatible polymer, and may for instance be a natural polymer or a synthetic polymer. In some embodiments, the polymer is a bioerodable polymer. Also, the polymer is preferably one which has little or no autofluorescence.

Preferably, however, the polymer comprises poly(L-lactide). Usually, the polymer is poly(L-lactide). Thus, the polymer may consist essentially of poly(L-lactide), or, for instance, the polymer may consist of poly(L-lactide). The poly(L-lactide) typically has a weight average molecular weight (M_{w}) of from 50,000 g/mol to 400,000 g/mol. Preferably, the poly(L-lactide) has a M_{w} of from 180,000 g/mol to 260,000 g/mol, for instance about 220 kg/mol, such as 221,000 g/mol.

A magnetic material, for instance as further defined hereinbefore, may optionally be included in the nanofibre precursor solution. Typically, the magnetic material comprises magnetic nanoparticles, which may for instance be iron oxide nanoparticles. The concentration of magnetic material in the nanofibre precursor solution is chosen to obtain a desired concentration of the magnetic material in the microscaffold end product. The concentration of magnetic material in the nanofibre precursor solution may for instance be from 0.1 to 2.0 weight % of the solution, for instance from 0.2 to 1.5 weight % of the solution. It may for instance be from 0.3 to 0.7 weight % of the solution, for example about 0.5 wt% of the solution.

Any suitable solvent may be employed in the nanofibre precursor solution. A wide range of solvents can be used in electrospinning, including for instance water and polar, nonpolar, protic and aprotic organic solvents. The solvent is chosen to suit the polymer or blend employed, particularly so that a homogeneous solution of the required concentration of the polymer can be obtained. HFIP is especially suitable when the polymer is poly(L-lactide).

Typically, therefore, the solvent is 1,1,1,3,3,3-Hexafluoroisopropanol (HFIP).

The concentration of the polymer in the solution should be high enough to achieve adequate chain entanglements in order for a continuous fibre to be formed. Typically, the concentration of the polymer in said solvent is from about 5 wt% to about 20 wt%. The concentration of the polymer in said solvent may for instance be from about 8 wt% to about 17 wt%. For instance, the concentration of the polymer in said solvent may be about 10wt%, to about 15wt%. For instance, the concentration of the polymer in said solvent may be about 13wt%.

The nanofibre precursor solution may be a solution of from 5wt% to 20wt% poly(L-lactide) in an organic solvent, such as HFIP. The nanofibre precursor solution may for instance be a solution of from 8wt% to 17wt% poly(L-lactide) in an organic solvent, such as HFIP. In one embodiment, the nanofibre precursor solution is a solution of about 10 wt % poly(L-lactide) in an organic solvent. The organic solvent is typically HFIP.

Typically, the dispensing capillary of the fibre forming module has an inner diameter of from about 0.5 mm to about 1.0 mm.

In order to ensure uniform deposition on the collection substrate, the electrospinning typically further comprises moving at least a portion of the fibre collection device relative to the fibre forming module during said deposition.

Thus, usually, the electrospinning further comprises moving at least a portion of the fibre collection device during said deposition.

Typically the fibre collection device comprises a rotatable portion. Usually, the electrospinning further comprises rotating the rotatable portion during said deposition. The rotatable portion is typically a rotatable drum. It is typically rotated at a rate of from about 80 rpm to about 120 rpm during the deposition.

Typically, therefore, the electrospinning further comprises rotating at least a portion of the fibre collection device during said deposition. Usually, the rotation is at a rate of from about 80 rpm to about 120 rpm.Usually, the fibre collection device comprises a rotatable drum and the electrospinning further comprises rotating said drum during said deposition. Typically, said rotation is at a rate of from about 80 rpm to about 120 rpm.

Deposition of the scaffold layer on the collection substrate is continued until a layer of a particular desired thickness has been obtained. Thus, typically the step of feeding said nanofibre precursor solution through the dispensing capillary whilst applying said voltage is performed until the thickness of said scaffold layer is from 10 µm to 200 µm. Often, the step of feeding said nanofibre precursor solution whilst applying said voltage is performed until the thickness is from 15 µm to 140 µm, or for instance from 20 µm to 120 µm, for example from 20 µm to 100 µm. The step of feeding said nanofibre precursor solution whilst applying said voltage may, for instance, be performed until the thickness is from 20 µm to 80 µm, for instance from 30 µm to 70 µm. The step may for instance be performed until the thickness is from 35 µm to 65 µm, or for example from 40 µm to 60 µm. It may for instance be performed until the thickness is about 50 µm. The term "about 50 µm" refers to 50 µm with a tolerance of ±10% (i.e. 50 µm ±5 µm).

Typically, the flow rate at which the nanofibre precursor solution is fed through the dispensing capillary is from 100 µl/hr to 3000 µl/hr. More typically, it is from 400 µl/hr to 2500 µl/hr, for instance about 2000 µl/hr.

The distance between the dispensing capillary and the collection substrate is typically from 200 mm to 400 mm. More typically, it is from 200 mm to 300 mm, for instance about 250 mm.

The voltage applied across the dispensing capillary and the fibre collection device is typically from 10 kV to 15 kV. More typically, it is from 12 kV to 13 kV, for instance about 12.5 kV.

Usually, the electrospinning is performed at a temperature of from 22 °C to 28 °C. More typically, the electrospinning is performed at a temperature of from 23 °C to 27 °C, for instance about 25 °C.

Typically, the electrospinning is performed in air having a relative humidity of from 20 % to 45 %. The electrospinning may for instance be performed in air having a relative humidity of 23 % to 40 %, for instance about 37 %.

The electrospinning process may further comprise: drying the scaffold layer thus produced. Typically, the scaffold layer is dried under vacuum. Typically the drying is done at room temperature.

Typically, the electrospinning process further comprises: removing the scaffold layer thus produced from the collection substrate. The collection substrate typically comprises a release paper sheet, aluminium foil, or a silicone-coated sheet.

In order to produce a microscaffold, the process further comprises: cutting the scaffold layer to produce a porous particle, which particle comprises said three dimensional network of fibres and has a particle size of less than or equal to 2000 µm.

Typically, the cutting is performed using a laser. The laser is typically an ultraviolet laser. The laser light may, for instance, have a wavelength of from 100 nm to 400 nm, or, for instance from 120 nm to 360 nm. In some embodiments the laser has a wavelength of from 180 nm to 210 nm. A 193 nm light laser may for instance be employed. A laser operating at a frequency of from 10 to 100 Hz may, for instance, be used, for instance a laser operating at 50 Hz.

In the process, the laser light is typically homogenised, to give a uniform beam (for instance a 10 mm × 10 mm beam) at a mask plane. The mask features allow a high resolution pattern to be imaged onto the scaffold layer, so that the scaffold layer may be cut by the laser in that particular pattern. Any suitable lens may be used to achieve this, but a 10X demagnification (X10 0. 15NA) lens may for instance be employed.

Accordingly, the process typically comprises directing laser light through a patterned mask onto the scaffold layer, and thereby cutting the scaffold layer into an array of microscaffolds. Typically, this comprises imaging a high resolution pattern of the laser onto the scaffold layer, and thereby cutting the scaffold layer into an array of microscaffolds. The mask may be designed so that microscaffolds have any desired shape and size. For instance, a hexagonal mask pattern may be employed in which the hexagons in the pattern have particular diameters, so that the microscaffolds produced by the process are in the shape of hexagonal prisms having approximately that diameter. However, any other polygonal pattern (such as a triangular, rectangular, square, pentagonal, heptagonal or octagonal pattern) may be employed to produce microscaffolds in the shapes of other polygonal prisms, or indeed a pattern of hollow circles could be employed to produce cylindrically-shaped microscaffolds.

Usually, during the step of cutting the scaffold layer with the laser, the scaffold layer is held at the image plane. Typically, it is held there between plates made of a material, for instance fused silica, which transmits the UV light and allows the majority of the surplus light (i.e. the light that machines through the scaffold) to escape in order to limit scaffold damage through reflections.

The focal length of the laser may be chosen to ensure optimal separation of the microscaffolds. Often, a focal length of from focus+200 µm to focus+50 µm, or a focal length of from focus-200 µm to focus-50 µm is employed. More typically, a focal length of from focus+180 µm to focus+80 µm, or a focal length of from focus-180 µm to focus-80 µm is employed. For instance a focal length of about focus+150 µm, about focus+100 µm, about focus-100 µm, or about focus-150 µm may be employed.

Often, a focal length of from focus+120 µm to focus+80 µm, or a focal length of from focus-120 µm to focus-80 µm is used, for instance a focal length of about focus+100 µm or about focus-100 µm.

The invention also provides a microscaffold which is obtainable by the process of the invention defined herein for producing a microscaffold.

The invention also provides a microscaffold which is obtained by the process of the invention defined herein for producing a microscaffold.

The invention further provides a composition which comprises a microscaffold of the invention.

Typically, the composition comprises a plurality of microscaffolds of the invention.

Each of the microscaffolds in said plurality may be as further defined above for the microscaffold of the invention. Also, each of the microscaffolds in said plurality may be the same. Thus, typically, the composition comprises a plurality of microscaffolds of the invention, each of which comprises a porous particle having the same shape. For instance, the composition may comprise a plurality of microscaffolds of the invention in which the porous particle has the shape of a hexagonal prism. More typically, the composition comprises a plurality of microscaffolds of the invention, each of which comprises a porous particle having the same shape and size, and more typically, having the same shape, size, and fibre diameter. Also, the polymer employed in each microscaffold in the plurality may be the same. The shape, size, fibre diameter and polymer of each microscaffold in the plurality may be as defined above for the microscaffold of the invention. Often, each of the microscaffolds in the plurality also further comprises a magnetic material, which, again, may be as further defined hereinbefore for the microscaffold of the invention.

Typically, the composition further comprises water. Thus, usually, the composition further comprises cell culture medium, which is, of course, aqueous.

Usually, the composition further comprises biological cells attached to the microscaffold or microscaffolds. The biological cells are typically mammalian cells. The composition typically also comprises extracellular matrix.

Thus, the microscaffold, or, when a plurality is present, at least one of the microscaffolds, or each of the microscaffolds, in the composition, may further comprise a cell culture within at least a portion of the microscaffold. The cell culture typically comprises biological cells, and typically also extracellular matrix. The cells are typically mammalian cells. The mammalian cells may for instance be liver cells (hepatocytes), kidney cells or nerve cells, for instance neuroblastoma cells. The cell culture is typically a three-dimensional (3D) cell culture.

Advantageously, cell-laden microscaffolds of the invention may be cryopreserved in freezing solution for substantial periods of time, and then thawed again before use, without adversely affecting cell viability or the ability of the cells to respond to chemical stimulation in a pharmacological manner. Cells cultures grown in the scaffolds may therefore be stored, in a frozen state, before they are employed by the end-user in drug testing or other applications, without adverse effect.

Accordingly, the composition of the invention as defined above may be in a frozen state. Thus, the composition of the invention as defined above, may be at a temperature at or below 0 °C, or for instance at a temperature at or below -5 °C, for example at a temperature at or below -10 °C.

More specifically, the composition of the invention may further comprise biological cells attached to the microscaffold or microscaffolds, and cell culture medium, and may be in a frozen state. It may for instance be frozen and at a temperature at or below 0 °C, or for instance at a temperature at or below -5 °C, for example at a temperature at or below -10 °C.

The invention also provides a multi-well assay plate, comprising:
a plurality of sample wells; and a composition, which comprises a microscaffold of the invention or a plurality of microscaffolds of the invention, in at least one of the sample wells. Usually, the composition of the invention is in at least 50% of the sample wells, for instance in at least 90% of the sample wells, if not in all of the sample wells.

The multi-well plate may for instance be a 96-well plate, a 384-well plate, a 1536-well plate, or a 3456-well plate, with a composition of the invention in at least one of the sample wells. Usually, the composition of the invention is in a plurality of the sample wells, for instance in at least 50% of the sample wells, for instance in at least 90% of the sample wells, if not in all of the sample wells.

Typically, in the multi-well assay plate of the invention, the composition of the invention which is in at least one of the sample wells, further comprise biological cells attached to the microscaffold or microscaffolds, and cell culture medium.

The composition of the invention, which is in the well or wells of the multi-well assay plate of the invention may itself be in a frozen state.

Thus, in the multi-well assay plate of the invention, the composition of the invention which is in at least one of the sample wells, typically further comprises biological cells (usually mammalian cells) attached to the microscaffold or microscaffolds in the composition, and cell culture medium, and may also be in a frozen state. The composition in the sample wells may for instance be frozen and at a temperature at or below 0 °C, or for instance at a temperature at or below -5 °C. It may for example be at a temperature at or below -10 °C.

Such a multi-well assay plate may be stored in the frozen state for future use, e.g for future use in screening compounds for biological use or drug screening.

The microscaffold of the invention is useful for drug screening and screening compounds for biological use, for instance when employed in a multi-well plate. In particular it is useful in high throughput drug screening using 3D cell cultures, and in high content screening using 3D cell cultures.

Herein also is provided the use not according to the invention of a microscaffold of the invention as defined above, a composition of the invention as defined above, or a multi-well assay plate of the invention as defined above, in: regenerative medicine, tissue engineering, screening compounds for biological use, or drug screening.

Typically, said drug screening is high throughput screening. In another embodiment, the drug screening is high content screening.

The scaffold is also useful in regenerative medicine (i.e. the process of replacing or regenerating human cells, tissues or organs to restore or establish normal function) and tissue engineering research. Accordingly, herein also is provided the use not according to the invention of the scaffold of the invention as defined above in regenerative medicine. Further provided is the use not according to the invention of the scaffold of the invention as defined above in tissue engineering.

The presence in the scaffold of a magnetic material increases the ease with which the microscaffolds may be maintained in suspension and manipulated externally. Such magnetic, cell-laden microscaffolds can be maintained in suspension by stirring using a standard magnetic stirring device. In addition, the magnetic microscaffolds may be manipulated using one or more external magnets, allowing for magnetic transfer from one location to another location, or indeed for retaining the microscaffolds in a particular position whilst the cell medium is renewed or replenished.

Thus, in a further aspect, the invention provides a method of manipulating one or more microscaffolds, which method comprises exposing a composition which comprises one or more microscaffolds of the invention as defined above which further comprise a magnetic material, to a magnetic field of a magnet, and thereby causing the one or more microscaffolds in the composition to be attracted to said magnet by magnetic attraction.

The magnet may be that of a magnetic stirrer, i.e. a laboratory device that employs a rotating magnetic field usually to cause a stir bar (also called "flea") immersed in a liquid to spin very quickly, thus stirring the liquid. The rotating field may be created either by a rotating magnet in the stirrer or a set of stationary electromagnets in the stirrer. In the method of the present invention, no stir bar need be employed because the microscaffolds themselves are magnetic and may therefore be suspended and stirred without the need of a rotating flea.

Thus, in one embodiment of the method of the invention, the composition which comprises the one or more microscaffolds further comprises a liquid medium, which for instance comprises cell culture medium, and said magnetic field of a magnet is a rotating magnetic field. Typically, exposing the composition to said rotating magnetic field aids suspension of the one or more microscaffolds in the liquid medium and/or the stirring of the liquid medium by the microscaffolds. This is illustrated by Figure 22.

In other embodiments of the method of the invention, an external magnet is used to manipulate the one or more magnetic microscaffolds, e.g. to effect transfer of the one or more microscaffolds from one location to another location, or to retain the one or more magnetic microscaffolds in a particular position, out of the way, whilst the cell medium is renewed or replenished. Thus, the method of the invention may further comprise one or more of the following steps:
- Altering, changing or renewing one or more components of the composition while the one or more microscaffolds are attracted to said magnet. For example, the magnet may be held at or near the edge of a vessel containing the composition (which vessel may be an individual sample well or receptacle, or indeed a multi-well plate) in order to attract the one or more microscaffolds to the edge of the vessel, and hold them against the edge of the vessel, so that a liquid supernatant comprising culture medium may be removed and replaced.
- Using the magnet to remove the one or more microscaffolds from the composition, and optionally to dispose the one or more microscaffolds at another location and/or into another composition. An example of this is shown in Fig. 23, which schematically illustrates the use of a magnet to remove magnetic microscaffolds from one sample well, move them above a different well, and then release them into that different well.
- Using the magnet to move the one or more microscaffolds in the composition from a first location in the composition to a second location in the composition.
- Using the magnet to retain the one or more microscaffolds at a particular location in the composition.

Typically in these embodiments of the method, the composition which comprises the one or more microscaffolds further comprises a liquid medium, for instance cell culture medium.

In these embodiments of the invention, the magnet employed to manipulate the microscaffolds may be a moveable within a magnet housing. Typically, both the magnet and the magnet housing are elongate, so that they may be introduced into a small sample well or similar vessel. Typically, the magnet is moveable into and out of a distal tip of the magnet housing. This allows the magnetic field to be brought into, or moved away from, the vicinity of a magnetic microscaffold which may be near the distal tip of the housing, either to effect attraction of the magnetic microscaffold to the distal tip, or release of the magnetic microscaffold from the distal tip. The use of such a device is described in Example 6 herein and shown schematically in Fig. 23.

In one embodiment, a plurality of such magnets which are moveable within a magnet housing are employed. The plurality of such magnets may be arranged to mirror the arrangement of wells in a multi-well assay plate. The magnets may therefore be used to move magnetic microscaffolds of the invention into and out of multiple wells in a multi-well assay plate at the same time, to facilitate efficient automation.

Thus, in one embodiment of the method of the invention, of manipulating one or more microscaffolds, the method comprises exposing a multi-well assay plate of the invention to the magnetic fields of a plurality of magnets arranged to mirror the arrangement of wells in the multi-well assay plate, and thereby causing the one or more microscaffolds in the composition in at least one of the sample wells to be attracted to a said magnet by magnetic attraction. Typically, each of the magnets in the plurality is an elongate magnet and is housed within an associated elongate magnet housing, and is moveable into and out of a distal tip of the associated elongate magnet housing. The magnets may be arranged in an array which mirrors the array of wells in the multi-well assay plate.

Typically in this embodiment the multi-well assay plate of the invention comprises a composition of the invention (comprising magnetic microscaffolds of the invention) in at least two of of the sample wells, and more typically in multiple sample wells of the multi-well assay plate, for instance in at least 50% of the sample wells of the multi-well assay plate, or in at least 90% of the sample wells of the multi-well assay plate, if not in all of the sample wells of the multi-well assay plate.

Bioinks, which contain cells and/or reagents, may be printed onto the microscaffolds of the invention. Thus, the microscaffolds of the invention may be used in combination with Bioinks to "spray" either cells or reagents onto scaffolds using appropriate inkjet devices. Dispensing of the cells on to the scaffolds may occur either during the production of the microscaffolds whilst they remain on the backing manufacturing material or when the scaffolds are in solution and can be attracted to the surface using magnetism. Cells that could be sprayed on to the scaffolds include cells of mammalian origin. Molecules and reagents that could be sprayed on to the surface include extracellular matrix molecules, for instance hyaluronidase, poly-D-lysine, laminin, vitronectin, fibronectin and collagen; peptides; nucleic acids, for instance clonal DNA; fluorescent dyes; streptavidin; and biotin.

Accordingly, the invention further provides a process for producing a microscaffold having cells and/or one or more reagents disposed on a surface thereof, which process comprises: disposing a bioink onto a surface of a microscaffold of the invention as defined herein, wherein the bioink comprises cells and/or one or more reagents.

The term "bioink" is known in the art. It is a material that behaves much like a liquid, allowing people to "print" it in order to create a desired shape. It is typically a liquid composition. When a bioink contains living cells, it is typically a liquid suspension of the cells. The cells may for instance be suspended in cell culture medium, alginate or phosphate-buffered saline (PBS). When a bioink comprises one or more reagents, the reagents may be dissolved or suspended in a solvent component of the bioink.

Thus, in the process of the invention, the bioink is typically a liquid composition which comprises the cells and/or one or more reagents. The bioink may further comprise a carrier suitable for cells or a solvent. The solvent typically comprises water. The carrier may be cell culture medium, alginate or phosphate-buffered saline (PBS).

The cells in the bioink may for instance be a cell line, stem cells or primary cells. The cells are typically mammalian cells. The mammalian cells may for instance be a cell line, stem cells or primary cells. The mammalian cells may for example be liver cells (hepatocytes), kidney cells, nerve cells, for instance neuroblastoma cells, fibroblasts, or keratinocytes.

The one or more reagents may for example be selected from extracellular matrix molecules, for instance hyaluronidase, poly-D-lysine, laminin, vitronectin, fibronectin and collagen; peptides; nucleic acids, for instance clonal DNA; fluorescent dyes; streptavidin; and biotin.

The step of disposing the bioink onto a surface of a microscaffold of the invention may be performed by a printer, for instance a 3D-printer. The printer may be an inkjet printer. The step of disposing the bioink onto a surface of a microscaffold may thus comprise printing the bioink onto said surface.

The microscaffold onto which the bioink is disposed may be as further defined anywhere herein for the microscaffold of the invention.

The microscaffolds themselves can form a component of a bioink. For example, bioinks comprising cell-loaded microscaffolds of the invention in a suitable carrier medium (e.g cell culture medium, alginate or PBS) can be used for bioprinting tissue-like materials into multi-well plates for cell based assays or used in more sophisticated 3D printing systems for the creation of more complex tissues and organs. A simple example of this would be to use two bioinks, one containing microscaffolds loaded with fibroblasts, one containing microscaffolds loaded with keratinocytes, and printing these as two layers to form a simplified skin model. The advantage of this approach rather than simply printing cells, is that the cells are already attached to a 3D support structure and have therefore adopted a 3D phenotype.

Accordingly, the invention further comprises a bioink, which bioink comprises a microscaffold of the invention as defined herein.

Usually, the microscaffold in the bioink further comprises cells attached to the microscaffold and, optionally, extracellular matrix.

The cells may be as further defined hereinbefore and may for instance be a cell line, stem cells or primary cells. The cells are typically mammalian cells. The mammalian cells may for instance be a cell line, stem cells or primary cells. The mammalian cells may for example be fibroblasts, keratinocytes, liver cells (hepatocytes), kidney cells or nerve cells, for instance neuroblastoma cells.

The bioink typically also therefore comprises a suitable carrier medium for the cells. Thus, the bioink typically further comprises cell culture medium, alginate or phosphate-buffered saline (PBS).

The invention also provides a method of providing a cell culture in one or more of the wells of a multi-well plate, the method comprising printing a bioink of the invention as defined above, in which cells are attached to the microscaffold, into one or more wells of a multi-well plate. The method may further comprise using the multi-well plate in drug screening.

The invention also provides a process for producing a tissue construct, which process comprises printing a bioink of the invention as defined above, in which cells are attached to the microscaffold. The step of printing the bioink of the invention typically comprisises printing the bioink into a desired shape. This is typically performed using a printer, which may for instance be an inkjet printer. The printer is generally a 3D printer.

In one embodiment, the process comprises printing a first bioink of the invention and printing a second bioink of the invention, wherein the cells attached to the microscaffold in the first bioink are different from the cells attached to the microscaffold in the second bioink. The cells in the first bioink may for instance be fibroblasts, and the cells in the second bioink may for instance be keratinocytes.

The present invention is further illustrated in the Examples which follow.

### EXAMPLES

### Example 1: microscaffold production

### Production of fibrous scaffolds by electrospinning

Poly (L-lactide) (PLLA) (Purasorb, PL 18, Corbion Purac, Netherlands) with a weight-averaged molecular weight (Mw) of 221 kg/mol was used in the manufacture of the material for microscaffolds. A stock solution containing 0.01 wt% of Rhodamine 6G in 1,1,1,3,3,3-hexafluoro-2-propanol (HFIP) (Sigma Aldrich, UK) was prepared. A solution containing 13 wt% polymer, 1wt% rhodamine stock solution and 86 wt% HFIP was prepared (resulting in a final 13 wt% polymer, 0.001 wt% rhodamine 6G and 86.999 wt% HFIP mix), with an additional amount of magnetic nanoparticles (Iron(II,III) oxide nanopowder, 50-100 nm particle size, Sigma Aldrich, UK) equivalent to 0.5 wt% of the solution.

The solutions were delivered at a constant feed rate of 2000 µl/h by a syringe pump and were electrospun vertically with an accelerating voltage of 12.5 kV supplied by a high voltage DC power supply. The solution was drawn from a 10 ml syringe connected to a blunt ended needle of internal diameter of 0.8 mm with PTFE tubing (inner diameter 1.6 mm for the first section, 1mm for the second section). Temperature and relative humidity were kept constant (respectively at 25 °C and 37 %RH) in a controlled atmosphere cabinet. Nonwoven fibrous scaffolds were collected on release paper sheets wrapped around an earthed rotating collector 25 cm from the tip of the needle. Longitudinal translation was also applied using a programmable motorised stage. 6.5 mL of solution were dispensed.

Fibre diameter and scaffold morphology were performed by scanning electronic microscopy (SEM) (Phenom G2 Pro equipped with Fibermetric software, Phenom World, Netherlands). The target average fibre diameter of the fibres was 4 µm with a tolerance of ±10% The target standard deviation around that average was below ±15% (for this particular material, inclusion of magnetic nanoparticles can increase standard deviation of the fibre diameters compared to standard material). Thickness of the sheet is measured using a micrometre. The target average thickness of the material was 50 µm with a tolerance of ±10%. The total dimensions of the sheet of material were about 250 mm by 300 mm of material within specifications for one run.

The fibrous mat was dried in a vacuum oven for over 48 hours at room temperature to reduce the amount of residual solvent remaining from the fabrication process. The sheet of material was then cut into smaller pieces for laser machining.

### Data for scaffolds produced by electrospinning

Figure 1 shows an SEM image (1000x magnification) of the bottom of a scaffold produced by electrospinning on 14 May 2015. Figure 2 shows an SEM image (1000x magnification) of the top of the same scaffold. The average fibre diameter measured at the bottom at the scaffold was: 3.89 ± 0.42 µm. The average fibre diameter measured at the top at the scaffold was: 3.92 ± 0.29 µm. The average thickness of the scaffold was: 50.3 ± 2 µm

### Production of microscaffolds by laser machining the electrospun scaffolds

Pulses of light at 193 nm were homogenised to give a uniform 10 mm × 10 mm beam at a mask plane. The mask features allowed a high resolution pattern to be imaged onto the electrospun scaffold with a 10X demagnification (X10 0.15NA lens). Other lenses and mask designs could also be used. The electrospun scaffold was held between fused silica plates that held the scaffold at the image plane and restricted the movement of the laser-cut features produced. The fused silica plates transmitted the UV light and allowed the majority of the surplus light (light that had machined through the scaffold) to escape in order to limit scaffold damage through reflections.

The laser employed operated with a frequency of 50 Hz. It machined each array of 56 hexagonal scaffolds for 2 seconds (100 pulses). Different focal lengths were evaluated before machining to ensure optimal separation of the scaffolds was obtained. Focal lengths of focus+150 µm, focus +100 µm, at focus, focus -100 µm, and focus -150 µm were evaluated. The scaffolds were imaged by SEM to evaluate machining. The chosen focal length was then used to manufacture a batch of scaffolds.

Thus, microscaffolds were produced by laser machining the electrospun scaffolds described above. Figure 3 shows an SEM image of microscaffolds produced by laser machining an electrospun scaffold at focus+100 µm. Figure 4 shows an SEM image of microscaffolds produced by laser machining an electrospun scaffold at focus-150 µm. Both the scaffolds machined at focus+100 µm and the scaffolds machined at focus-150 µm were used in subsequent trials.

### Example 2: successful attachment of mammalian cells to the microscaffolds

The following three mammalian cell types were successfully attached to microscaffolds of the invention: (i) HepG2 hepatocytes (liver cells); (ii) HEK293 kidney cells, and (iii) SH-SY5Y neuroblastoma (nerve cells). Cells previously grown in 2-D were isolated from a cell culture flask using accutase treatment followed by media addition and counting the concentration of cells per millilitre. Cells were either mixed with microscaffolds in a 15 ml or 50 ml conical tube, mixing the content every 30 minutes for a number of hours (up to 4) then dispensed into microplates or cells and microscaffolds were dispensed into commercially available microwell plates that have been treated to prevent the cells adhering to the plate and hence encouraging them to adhere to the microscaffolds. These cells/microscaffolds were incubated in these well plates to encourage adhesion and growth over a number of hours. Figures 5, 6 and 7 are light microscope images of these three mammalian cell types, respectively, adhered to a number of 3D scaffolds after 4 days' growth.

### Example 3: cell viability of cells attached to the microscaffolds

### Standard, non-magnetic microscaffolds

A commercially available kit - a Nano-Luc technology kit - was used to determine cell viability *in vitro.* The Nano-Luc technology kit is based on the conversion of a substrate in live cells to a product resulting in light emission. The amount of light (luminescence) generated is proportional to metabolism of viable cells. Figures 8, 9 and 10 are plots of luminescence (in relative light units, RLU) on the y axis versus the number of microscaffolds (within the wells of a microwell plate) on the x axis. Figure 8 presents the results of the experiment in which HepG2 cells were attached to the microscaffolds, Figure 9 presents the results of the experiment in which HEK293 cells were attached to microscaffolds, and Figure 10 presents the results of the experiment in which SH-SY5Y cells were attached to microscaffolds. In all cases the results show that scaffold-adherent cells were alive, viable and metabolising the substrate to generate luminescence (light). The amount of light generated is proportional to the number of scaffolds and therefore the number of viable cells within the wells of the microwell plate.

### Magnetic microscaffolds versus standard (non-magnetic) microscaffolds

The same cell viability assay showed that HepG2 cells plated on magnetic microscaffolds have comparable viability and metabolism to those grown on standard (non-magnetic) microscaffolds. Figure 11 is a plot of the number of microscaffolds (within the wells of a microwell plate) on the x axis, versus the luminescence (in relative light units, RLU) on the y axis generated by HepG2 cells when attached to standard microscaffolds of the invention (upper plot) or magnetic microscaffolds of the invention (lower plot). The data indicate that the incorporation of magnetic material - in this case 1% by weight iron (II,III) oxide - into the microscaffold material is not toxic to cells.

### Example 4: functional pharmacology on microscaffolds

HEK293 cells expressing a sequence of DNA that is stimulated by increases in cyclic AMP were incubated with a compound (forskolin) that generates a dose-dependent increase in cAMP. Cells grown on a two-dimensional (2-D) surface (as opposed to on a 3-D microscaffold of the invention) were compared with cells grown on standard (non-magnetic) microscaffolds of the invention (see Fig. 12) and magnetic microscaffolds of the invention (see Fig. 13). Data were substantially identical between 2-D, 3-D non-magnetic microscaffolds and 3-D magnetic microscaffolds, demonstrating that cells can grow on magnetic as well as non-magnetic 3-D microscaffolds of the invention, and can respond in a pharmacological manner to chemical stimulation in a way comparable to cells grown on a 2-D surface.

### Example 5: cryopreservation of cell-laden microscaffolds

HEK293 cells were cryo-preserved on either non-magnetic ("standard") or magnetic microscaffolds of the invention, in freezing solution, and then recovered and dispensed into well plates and assayed for cAMP activity following forskolin treatment, after 24, 48 or 72 hours of cryopreservation. For cryo-preservation, microscaffolds impregnated with cells were allowed to settle under gravity (non-magnetic scaffolds) or using magnetism (iron containing scaffolds) in a 15 ml conical tube and most of the media removed with a pipette. One millilitre of solution containing 90% foetal bovine serum and 10% dimethyl sulphoxide was used to dilute the microscaffolds then pipetted into a cryo-preservation tube. This was placed into a cryo-preservation device (known as a "Mr Frosty") containing 100% isopropanol and placed into a -80 degree centrigrade freezer. After 24 hours the vial was transferred into the vapour phase of a liquid nitrogen storage unit. Control experiments without cryopreservation were performed on standard and magnetic microscaffolds of the invention. The results are shown in Figures 14 to 21, all of which plot the % maximal response (normalised to the number of scaffolds), to chemical stimulation by forskolin, of cells adhered to either standard or magnetic microscaffolds of the invention (y axis) versus the base 10 logarithm of the concentration of forskolin in units of µM (x axis). Figures 14 and 15 relate to non-cryopreserved cells on standard and magnetic microscaffolds respectively. Figures 16 and 17 relate to cells, on standard and magnetic microscaffolds respectively, that had previously been cryopreserved in freezing solution for 24 hours whilst on the scaffolds. Figures 18 and 19 relate to cells, on standard and magnetic microscaffolds respectively, that had previously been cryopreserved in freezing solution for 48 hours whilst on the scaffolds. Figures 20 and 21 relate to cells, on standard and magnetic microscaffolds respectively, that had previously been cryopreserved in freezing solution for 72 hours whilst on the scaffolds. The results in the Figures demonstrate that cells that have been cryopreserved for up to 72 hours can be sustained on both standard and magnetic scaffolds and respond in a pharmacological manner to chemical stimulation in a way comparable to cells that have not been cryopreserved.

### Example 6: magnetic manipulation of microscaffolds

The magnetic microscaffolds of the invention are easy to manipulate using an external magnet. Microscaffolds laden with cells can be manipulated magnetically during their use, facilitating the handling of cells and the use of the cell-laden microscaffolds in cell-based assays. Figure 22 shows freeze frames taken from a video which shows the ease with which magnetic microscaffolds of the invention may be handled, and in particular the ease with which they can be suspended in an aqueous medium by constant stirring using an external magnetic stirring device. By mixing the solution constantly one can aspirate aliquots of liquid from the vessel and dispense them into micro-well plates using either bulk reagent pipetting devices or tip based dispensing devices.

Figure 23 then shows how an external magnet, in particular a thin bar magnet placed inside a sealed pipette tip, can be used to facilitate discrete well-to-well transfer. The device can be used to attract microscaffolds from the inside of a well in a multi-well assay plate onto the outside of the pipette tip, so that they can then be removed from the well and transferred into another well (either in the same plate or in a different multi-well assay plate) or released back into the same well after its contents have been changed or renewed. In order to release the microscaffolds from the outside of the pipette tip and into the desired well, the bar magnet is lifted out from inside the sealed pipette tip, so that the magnetic field is removed from the vicinity of the microscaffolds and ceases to attract the microscaffolds to the pipette tip.

Figure 23 shows the device as a single pipette, but the device can instead have multiple pipette tips, for instance 8, 12, 16, 96, 384 or 1536 pipette tips/pins, arranged to mirror the arrangement of wells in a multi-well plate. Thus, similar devices having 8, 12, 16, 96, 384 or 1536 pipette tips can be employed, in which the tips are spaced apart at intervals and arranged in an array which mirrors the arrangement of wells in a standard multi-well assay plate. Such devices can be used to lift many samples of magnetic microscaffolds of the invention out of different wells in a multi-well plate at the same time, and then, for instance, place the many microscaffold samples back into those wells (e.g. after their contents have been changed or replenished) or transfer them into the wells of a different multi-well plate. This kind of manipulation facilitates the rapid handling of many cell-laden scaffold samples at the same time, and is useful in, e.g., high-throughput drug-screening assays in which drug candidates are tested on 3D cell samples grown on the microscaffolds of the invention.

## Claims

1. A microscaffold comprising a porous particle, which particle:
comprises a three dimensional network of electrospun fibres, which fibres comprise a biocompatible polymer,
wherein the particle has the shape of a cylinder or a polygonal prism, wherein the diameter of the cylinder or polygonal prism is from 20 µm to 2000 µm, and wherein the height of the cylinder or polygonal prism is from 10 µm to 200 µm.

2. A microscaffold according to claim 1 wherein the porosity is greater than 75%, preferably greater than 80%, even more preferably greater than 90%.

3. A microscaffold according to any one of the preceding claims wherein the mean diameter of the fibres is from 500 nm to 10 µm, preferably from 2 µm to 6 µm.

4. A microscaffold according to claim 3 wherein the relative standard deviation from said mean is less than or equal to 25%.

5. A microscaffold according to any of the preceding claims wherein the diameter of the cylinder or polygonal prism is from 40 µm to 400 µm.

6. A microscaffold according to any of the preceding claims wherein
the height of the cylinder or polygonal prism is from 20 µm to 80 µm.

7. A microscaffold according to any preceding claim wherein the particle has the shape of a triangular prism, a tetragonal prism, a pentagonal prism, a hexagonal prism, a heptagonal prism, an octagonal prism, an enneagonal prism or a decagonal prism, preferably a hexagonal prism.

8. A microscaffold according to any one of the preceding claims wherein the particle further comprises a magnetic material, preferably a paramagnetic, ferromagnetic, ferrimagnetic or superparamagnetic material; even more preferably
wherein the fibres further comprise said magnetic material, and/or
wherein the magnetic material comprises iron (II, III) oxide.

9. A microscaffold according to any one of the preceding claims wherein the fibres further comprise one or more of: an extracellular matrix molecule, a peptide, a nucleic acid, a fluorescent dye, streptavidin, and biotin,
optionally wherein the extracellular matrix molecule is hyaluronidase, d-lysine, laminin, vitronectin, fibronectin or collagen, and
optionally wherein the nucleic acid is clonal DNA.

10. A microscaffold according to any one of the preceding claims wherein the fibres comprise any of:
poly(lactide); poly(glycolic acid); poly(ε-caprolactone); polyhydroxybutyrate; polystyrene; polyethylene; polypropylene; poly(ethylene oxide); a poly(ester urethane); poly(vinyl alcohol); polyacrylonitrile; polylactide; polyglycolide; polyurethane; polycarbonate; polyimide; polyamide; aliphatic polyamide; aromatic polyamide; polybenzimidazole; poly(ethylene terephthalate); poly[ethylene-co-(vinyl acetate)]; poly(vinyl chloride); poly(methyl methacrylate); poly(vinyl butyral); poly(vinylidene fluoride); poly(vinylidene fluoride-co-hexafluoropropylene); cellulose acetate; poly(vinyl acetate); poly(acrylic acid); poly(methacrylic acid); polyacrylamide; polyvinylpyrrolidone; poly(phenylene sulfide); hydroxypropylcellulose; polyvinylidene chloride, polytetrafluoroethylene, a polyacrylate, a polymethacrylate, a polyester, a polysulfone, a polyolefin, polysilsesquioxane, silicone, epoxy, cyanate ester, a bis-maleimide polymer; polyketone, polyether, polyamine, polyphosphazene, polysulfide, an organic/inorganic hybrid polymer, a copolymer thereof, or a blend thereof; and/or may comprise any of poly(glycolic acid); polyhydroxybutyrate; and poly(ester urethanes); preferably wherein the fibres comprise poly(L-lactide).

11. A composition which comprises a microscaffold as defined in any one of the preceding claims or a plurality of microscaffolds as defined in any one of the preceding claims; preferably wherein the composition
further comprises a cell culture medium; and/or wherein the composition further comprises mammalian cells attached to the microscaffold or microscaffolds and, optionally, extracellular matrix;
optionally wherein the composition is frozen.

12. A multi-well assay plate comprising:
a plurality of sample wells; and
a composition as defined in claim 11 in at least one of the sample wells.

13. Use of a microscaffold as defined in any one of claims 1 to 10, a composition as defined in claim 11, or a multi-well assay plate as defined in claim 12, in, tissue engineering, screening compounds for biological use or drug screening.

14. A method of manipulating one or more microscaffolds, which method comprises exposing a composition which comprises one or more microscaffolds as defined in claim 8 to a magnetic field of a magnet, and thereby causing the one or more microscaffolds in the composition to be attracted to said magnet by magnetic attraction, preferably wherein the method
further comprises:
(i) altering, changing or renewing one or more components of the composition while the one or more microscaffolds are attracted to said magnet; or
(ii) using the magnet to remove the one or more microscaffolds from the composition, and optionally to dispose the one or more microscaffolds at another location and/or into another composition; or
(iii) using the magnet to move the one or more microscaffolds in the composition from a first location in the composition to a second location in the composition; or
(iv) using the magnet to retain the one or more microscaffolds at a particular location in the composition.

15. A process for producing a microscaffold, which process comprises:
(a)producing a scaffold layer, which scaffold layer comprises a porous, three dimensional network of electrospun fibres, wherein the fibres comprises a biocompatible polymer; and
(b) cutting the scaffold layer to produce a porous particle, which particle comprises said three dimensional network of fibres,
wherein the particle is cut such that it has the shape of cylinder or a polygonal prism, wherein the diameter of the cylinder or polygonal prism is from 20 µm to 2000 µm, and wherein the height of the cylinder or polygonal prism is from 10 µm to 200 µm, preferably wherein the microscaffold has any of the features of claims 2-10.

16. A process according to claim 15, wherein the cutting is performed using a laser, preferably an ultraviolet laser, even more preferably wherein the laser has a wavelength of from 100nm to 400nm, for instance, from 120 to 360nm or 180 to 210nm.

17. A process according to claim 16 wherein the laser has a frequency of from 10 to 100Hz, and/or wherein the process comprises directing laser light through a patterned mask onto the scaffold layer, and thereby cutting the scaffold layer into an array of microscaffolds.

## Patentansprüche

1. Mikrogerüst umfassend ein poröses Partikel, welches Partikel:
ein dreidimensionales Netzwerk von elektrogesponnenen Fasern umfasst, welche Fasern ein biokompatibles Polymer umfassen,
wobei das Partikel die Gestalt eines Zylinders oder eines polygonalen Prismas aufweist, wobei der Durchmesser des Zylinders oder polygonalen Prismas 20 µm bis 2000 µm beträgt und wobei die Höhe des Zylinders oder polygonalen Prismas 10 µm bis 200 µm beträgt.

2. Mikrogerüst nach Anspruch 1, wobei die Porosität höher als 75 %, bevorzugt höher als 80 %, sogar noch bevorzugter höher als 90 % ist.

3. Mikrogerüst nach einem der vorhergehenden Ansprüche, wobei der mittlere Durchmesser der Fasern 500 nm bis 10 µm, bevorzugt 2 µm bis 6 µm beträgt.

4. Mikrogerüst nach Anspruch 3, wobei die relative Standardabweichung von dem Mittelwert geringer als oder gleich 25 % ist.

5. Mikrogerüst nach einem der vorhergehenden Ansprüche, wobei der Durchmesser des Zylinders oder polygonalen Prismas 40 µm bis 400 µm beträgt.

6. Mikrogerüst nach einem der vorhergehenden Ansprüche, wobei
die Höhe des Zylinders oder polygonalen Prismas 20 µm bis 80 µm beträgt.

7. Mikrogerüst nach einem der vorhergehenden Ansprüche, wobei das Partikel die Gestalt eines dreieckigen Prismas, eines viereckigen Prismas, eines fünfeckigen Prismas, eines sechseckigen Prismas, eines siebeneckigen Prismas, eines achteckigen Prismas, eines neuneckigen Prismas oder eines zehneckigen Prismas, bevorzugt eines sechseckigen Prismas aufweist.

8. Mikrogerüst nach einem der vorhergehenden Ansprüche, wobei das Partikel ferner ein magnetisches Material, bevorzugt ein paramagnetisches, ferromagnetisches, ferrimagnetisches oder superparamagnetisches Material umfasst, wobei sogar noch bevorzugter die Fasern ferner das magnetische Material umfassen und/oder wobei das magnetische Material Eisen(II, III)Oxid umfasst.

9. Mikrogerüst nach einem der vorhergehenden Ansprüche, wobei die Fasern ferner eines oder mehrere umfassen von: einem extrazellulären Matrixmolekül, einem Peptid, einer Nukleinsäure, einem fluoreszierenden Farbstoff, Streptavidin und Biotin,
wobei wahlweise das extrazelluläre Matrixmolekül Hyaluronidase, d-Lysin, Laminin, Vitronectin, Fibronectin oder Kollagen ist und
wobei wahlweise die Nukleinsäure klonale DNA ist.

10. Mikrogerüst nach einem der vorhergehenden Ansprüche, wobei die Fasern irgendeines umfassen von:
Poly(lactid), Poly(glykolsäure, Poly(ε-caprolacton), Polyhydroxybutyrat, Polystyrol, Polyethylen, Polypropylen, Poly(ethylenoxid, einem Poly(esterurethan), Poly(vinylalkohol), Polyacrylnitril, Polylactid, Polyglykolid, Polyurethan, Polycarbonat, Polyimid, Polyamid, aliphatischem Polyamid, aromatischem Polyamid, Polybenzimidazol, Poly(ethylenterephthalat), Poly[ethylen-co-(vinylacetat)], Poly(vinylchlorid), Poly)methylmethacrylat, Poly(vinylbutyral), Poly(vinylidenfluorid), Poly(vinylidenfluorid-co-hexafluorpropylen), Celluloseacetat, Poly(vinylacetat), Poly(acrylsäure), Poly(methacrylsäure), Polyacrylamid, Polyvinylpyrrolidon, Poly(phenylensulfid), Hydroxypropylcellulose, Polyvinylidenchlorid, Polytetrafluorethylen, einem Polyacrylat, einem Polymethacrylat, einem Polyester, einem Polysulfon, einem Polyolefin, Polysilsequioxan, Silicon, Epoxy, Cyanatester, einem Bismaleimidpolymer, Polyketon, Polyether, Polyamin, Polyphosphazen, Polysulfid, einem organischen/anorganischen Hybridpolymer, einem Copolymer davon oder einer Abmischung davon; und/oder irgendeines von Poly(glykolsäure), Polyhydroxybutyrat und Poly(esterurethan) umfassen können, wobei die Faser bevorzugt Poly(L-lactid umfasst.

11. Zusammensetzung, die ein Mikrogerüst, wie in irgendeinem der vorhergehenden Ansprüche definiert, oder eine Mehrzahl von Mikrogerüsten, wie in einem der vorhergehenden Ansprüche definiert, umfasst, wobei bevorzugt die Zusammensetzung ferner ein Zellkulturmedium umfasst; und/oder wobei die Zusammensetzung ferner Säugerzellen, die an das Mikrogerüst oder Mikrogerüste angebracht sind, und wahlweise extrazelluläre Matrix umfasst;
wobei wahlweise die Zusammensetzung gefroren ist.

12. Assay-Platte von mehreren Vertiefungen, umfassend:
eine Mehrzahl von Probevertiefungen; und
eine Zusammensetzung, wie in Anspruch 11 definiert, in mindestens einer der Probevertiefungen.

13. Verwendung eines Mikrogerüsts, wie in einem der Ansprüche 1 bis 10 definiert, einer Zusammensetzung, wie in Anspruch 11 definiert oder Assay-Platte von mehreren Vertiefungen, wie in Anspruch 12 definiert, beim Gewebe-Engineering, in Screening-Verbindungen für biologische Verwendung oder Drogenscreening.

14. Verfahren zum Manipulieren eines oder mehrerer Mikrogerüste, welches Verfahren das Aussetzen einer Zusammensetzung, die ein oder mehrere Mikrogerüste, wie in Anspruch 8 definiert, umfasst, einem Magnetfeld eines Magnets und dadurch Verursachen umfasst, dass das eine oder die mehreren Mikrogerüste in der Zusammensetzung an den Magnet durch magnetische Anziehung angezogen werden, wobei bevorzugt das Verfahren ferner Folgendes umfasst:
(i) Ändern, Auswechseln oder Erneuern einer oder mehrerer Komponenten der Zusammensetzung, während das eine oder die mehreren Mikrogerüste an den Magnet angezogen werden; oder
(ii) Verwenden des Magnets zum Entfernen des einen oder der mehreren Mikrogerüste aus der Zusammensetzung und wahlweise zum Absetzen des einen oder der mehreren Mikrogerüsts an einer anderen Stelle und/oder in eine andere Zusammensetzung; oder
(iii) Verwenden des Magnets zum Bewegen des einen oder der mehreren Mikrogerüste in der Zusammensetzung von einer ersten Stelle in der Zusammensetzung an eine zweite Stelle in der Zusammensetzung; oder
(iv) Verwenden des Magnets zum Halten des einen oder der mehreren Mikrogerüste an einer spezifischen Stelle in der Zusammensetzung.

15. Verfahren zum Herstellen eines Mikrogerüsts, welches Verfahren Folgendes umfasst:
(a) Herstellen einer Gerüstschicht, welche Gerüstschicht ein poröses, dreidimensionales Netzwerk von elektrogesponnenen Fasern umfasst, wobei die Fasern ein biokompatibles Polymer umfassen; und
(b) Schneiden der Gerüstschicht, um ein poröses Partikel herzustellen, welches Partikel das dreidimensionale Netzwerk von Fasern umfasst,
wobei das Partikel so geschnitten wird, dass es die Gestalt eines Zylinders oder eines polygonalen Prismas aufweist, wobei der Durchmesser des Zylinders oder polygonalen Prismas 20 µm bis 2000 µm beträgt und wobei die Höhe des Zylinders oder polygonalen Prismas 10 µm bis 200 µm beträgt, wobei bevorzugt das Mikrogerüst irgendeines der Merkmale der Ansprüche 2-10 aufweist.

16. Verfahren nach Anspruch 15, wobei das Schneiden unter Anwendung eines Lasers, bevorzugt eines Ultraviolettlasers, ausgeführt wird, wobei selbst noch bevorzugter der Laser eine Wellenlänge von 100 nm bis 400 nm, beispielsweise von 120 bis 360 nm oder 180 bis 210 nm aufweist.

17. Verfahren nach Anspruch 16, wobei der Laser eine Frequenz von 10 bis 100 Hz aufweist und/oder wobei das Verfahren das Richten von Laserlicht durch eine gemusterte Maske auf die Gerüstschicht und dadurch Schneiden der Gerüstschicht in eine Anordnung von Mikrogerüsten umfasst.

## Revendications

1. Microéchafaudage comprenant une particule poreuse, laquelle particule :
comprend un réseau tridimensionnel de fibres électrofilées, lesquelles fibres comprennent un polymère biocompatible,
dans lequel la particule a la forme d'un cylindre ou d'un prisme polygonal, dans lequel le diamètre du cylindre ou du prisme polygonal est de 20 µm à 2000 µm, et dans lequel la hauteur du cylindre ou du prisme polygonal est de 10 µm à 200 µm.

2. Microéchafaudage selon la revendication 1 dans lequel la porosité est supérieure à 75 %, de préférence supérieure à 80 %, encore plus préférablement supérieure à 90 %.

3. Microéchafaudage selon l'une quelconque des revendications précédentes dans lequel le diamètre moyen des fibres est de 500 nm à 10 µm, de préférence de 2 µm à 6 µm.

4. Microéchafaudage selon la revendication 3 dans lequel l'écart standard relatif par rapport à ladite moyenne est inférieur ou égal à 25 %.

5. Microéchafaudage selon l'une quelconque des revendications précédentes dans lequel le diamètre du cylindre ou du prisme polygonal est de 40 µm à 400 µm.

6. Microéchafaudage selon l'une quelconque des revendications précédentes dans lequel
la hauteur du cylindre ou du prisme polygonal est de 20 µm à 80 µm.

7. Microéchafaudage selon une quelconque revendication précédente dans lequel la particule a la forme d'un prisme triangulaire, d'un prisme tétragonal, d'un prisme pentagonal, d'un prisme hexagonal, d'un prisme heptagonal, d'un prisme octogonal, d'un prisme ennéagonal ou d'un prisme décagonal, de préférence d'un prisme hexagonal.

8. Microéchafaudage selon l'une quelconque des revendications précédentes dans lequel la particule comprend en outre un matériau magnétique, de préférence un matériau paramagnétique, ferromagnétique, ferrimagnétique ou superparamagnétique ; encore plus préférablement
dans lequel les fibres comprennent en outre ledit matériau magnétique, et/ou
dans lequel le matériau magnétique comprend de l'oxyde de fer (II, III).

9. Microéchafaudage selon l'une quelconque des revendications précédentes dans lequel les fibres comprennent en outre un ou plusieurs parmi : une molécule de matrice extracellulaire, un peptide, un acide nucléique, un colorant fluorescent, streptavidine et biotine, optionnellement dans lequel la molécule de matrice extracellulaire est hyaluronidase, d-lysine, laminine, vitronectine, fibronectine ou collagène, et optionnellement dans lequel l'acide nucléique est un ADN clonal.

10. Microéchafaudage selon l'une quelconque des revendications précédentes dans lequel les fibres comprennent l'un quelconque parmi :
polylactide ; acide polyglycolique ; poly-e-caprolactone ; polyhydroxybutyrate ; polystyrène ; polyéthylène ; polypropylène ; polyoxyde d'éthylène ; polyesteruréthane ; alcool polyvinylique ; polyacrylonitrile ; acide polylactique ; acide polyglycolique ; polyuréthane ; polycarbonate ; polyimide ; polyamide ; polyamide aliphatique ; polyamide aromatique ; polybenzimidazol ; polytéréphtalate d'éthylène ; copolymère d'acétate de vinyle-éthylène ; polychlorure de vinyle ; polyméthacrylate de méthyle ; polybutyral de vinyle ; polyfluorure de vinylidène ; copolymère d'hexafluoropropylène et fluorure de vinylidène ; acétate de cellulose ; acétate de polyvinyle ; acide polyacrylique ; acide polyméthacrylique ; polyacrylamide ; polyvinylpyrrolidone ; polysulfure de phénylène ; hydroxypropyl cellulose ; polychlorure de vinylidène, polytétrafluoroéthylène, un polyacrylate, un polyméthacrylate, un polyester, une polysulfone, une polyoléfine, polysilsesquioxane, silicone, époxy, ester de cyanate, un polymère de bismaléimide ; polycétone, polyéther, polyamine, polyphosphazène, polysulfure, un polymère hybride organique/inorganique, un copolymère de ceux-ci ou un mélange de ceux-ci ; et/ou peuvent comprendre l'un quelconque parmi acide polyglycolique ; polyhydroxybutyrate ; et polyesteruréthanes ; de préférence dans lequel les fibres comprennent un polymère L-lactide.

11. Composition qui comprend un microéchafaudage tel que défini dans l'une quelconque des revendications précédentes ou une pluralité de microéchafaudages tels que définis dans l'une quelconque des revendications précédentes ; de préférence dans laquelle la composition comprend en outre un milieu de culture cellulaire ; et/ou dans laquelle la composition comprend en outre des cellules de mammifère fixées au microéchafaudage ou aux microéchafaudages et, optionnellement, une matrice extracellulaire ;
optionnellement dans laquelle la composition est congelée.

12. Plaque d'essai à puits multiples comprenant :
une pluralité de puits d'échantillons ; et
une composition telle que définie dans la revendication 11 dans au moins un des puits d'échantillons.

13. Utilisation d'un microéchafaudage tel que défini dans l'une quelconque des revendications 1 à 10, d'une composition telle que définie dans la revendication 11, ou d'une plaque d'essai à puits multiples telle que définie dans la revendication 12, dans l'ingénierie tissulaire, le criblage de composés pour une utilisation biologique ou le criblage de médicaments.

14. Procédé de manipulation d'un ou plusieurs microéchafaudages, lequel procédé comprend l'exposition d'une composition qui comprend un ou plusieurs microéchafaudages tels que définis dans la revendication 8 à un champ magnétique d'un aimant, et ainsi le fait d'amener les un ou plusieurs microéchafaudages dans la composition à être attirés vers ledit aimant par attraction magnétique, de préférence dans lequel le procédé comprend en outre :
(i) la modification, le changement ou le renouvellement d'un ou plusieurs composés de la composition tandis que les un ou plusieurs microéchafaudages sont attirés vers ledit aimant ; ou
(ii) l'utilisation de l'aimant pour retirer les un ou plusieurs microéchafaudages de la composition, et optionnellement pour disposer les un ou plusieurs microéchafaudages à un autre emplacement et/ou dans une autre composition ; ou
(iii) l'utilisation de l'aimant pour déplacer les un ou plusieurs microéchafaudages dans la composition d'un premier emplacement dans la composition à un second emplacement dans la composition ; ou
(iv) l'utilisation de l'aimant pour retenir les un ou plusieurs microéchafaudages à un emplacement particulier dans la composition.

15. Procédé pour produire un microéchafaudage, lequel procédé comprend :
(a) la production d'une couche d'échafaudage, laquelle couche d'échafaudage comprend un réseau poreux tridimensionnel de fibres électrofilées, dans lequel les fibres comprennent un polymère biocompatible ; et
(b) la découpe de la couche d'échafaudage pour produire une particule poreuse, laquelle particule comprend ledit réseau tridimensionnel de fibres,
dans lequel la particule est découpée de sorte qu'elle ait la forme d'un cylindre ou d'un prisme polygonal, dans lequel le diamètre du cylindre ou du prisme polygonal est de 20 µm à 2000 µm, et dans lequel la hauteur du cylindre ou du prisme polygonal est de 10 µm à 200 µm, de préférence dans lequel le microéchafaudage a l'une quelconque des caractéristiques selon les revendications 2 à 10.

16. Procédé selon la revendication 15, dans lequel la découpe est réalisée en utilisant un laser, de préférence un laser ultraviolet, encore plus préférablement dans lequel le laser a une longueur d'onde de 100 nm à 400 nm, par exemple, de 120 à 360 nm ou 180 à 210 nm.

17. Procédé selon la revendication 16 dans lequel le laser a une fréquence de 10 à 100 Hz, et/ou dans lequel le procédé comprend la direction d'une lumière laser à travers un masque à motifs sur la couche d'échafaudage, et ainsi la découpe de la couche d'échafaudage en une série de microéchafaudages.
